(19) **Europäisches Patentamt / European Patent Office / Office européen des brevets**

(11) **EP 2 076 542 B1**

(12) **FASCICULE DE BREVET EUROPEEN**

(45) Date de publication et mention
de la délivrance du brevet:
**15.08.2012 Bulletin 2012/33**

(21) Numéro de dépôt: **07821118.2**

(22) Date de dépôt: **10.10.2007**

(51) Int Cl.:
*C07K 16/30* (2006.01)    *C12N 15/13* (2006.01)
*C12N 15/63* (2006.01)    *C12P 21/08* (2006.01)
*G01N 33/577* (2006.01)    *G01N 33/574* (2006.01)
*A61K 39/395* (2006.01)    *A61K 47/48* (2006.01)
*A61P 35/00* (2006.01)

(86) Numéro de dépôt international:
**PCT/EP2007/060750**

(87) Numéro de publication internationale:
**WO 2008/043777 (17.04.2008 Gazette 2008/16)**

(54) **UTILISATION D'ANTICORPS MONOCLONAUX SPÉCIFIQUES DE LA FORME O-ACÉTYLÉE DU GANGLIOSIDE GD2 DANS LE TRAITEMENT DE CERTAINS CANCERS**

VERWENDUNG VON FÜR DIE O-ACETYLIERTE FORM VON GD2-GANGLIOSID SPEZIFISCHEN MONOKLONALEN ANTIKÖRPERN ZUR BEHANDLUNG BESTIMMTER KREBSARTEN

USE OF MONOCLONAL ANTIBODIES SPECIFIC TO THE O-ACETYLATED FORM OF GD2 GANGLIOSIDE FOR THE TREATMENT OF CERTAIN CANCERS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **10.10.2006 FR 0608881**

(43) Date de publication de la demande:
**08.07.2009 Bulletin 2009/28**

(73) Titulaire: **Université de Nantes**
**44035 Nantes Cedex 1 (FR)**

(72) Inventeurs:
• **BIRKLE, Stéphane**
**F-44000 Nantes (FR)**
• **MUSSINI, Jean-Marie**
**F-44200 Nantes (FR)**
• **AUBRY, Jacques**
**F-44000 Nantes (FR)**
• **BARBET, Jacques**
**F-44000 Nantes (FR)**
• **CHATAL, Jean-François**
**F-44380 Pornichet (FR)**
• **MEZAZIGH HAMI, Assia**
**44300 Nantes (FR)**
• **SUPIOT, Stéphane**
**44000 Nantes (FR)**

(74) Mandataire: **Barbot, Willy**
**SIMODORO**
**Parc Cézanne II, Bât. G**
**290 Avenue Galilée**
**13857 Aix en Provence Cedex 3 (FR)**

(56) Documents cités:
• **CERATO E ET AL: "Variable region gene segments of nine monoclonal antibodies specific to disialogangliosides (GD2, GD3) and their O-acetylated derivatives." HYBRIDOMA AUG 1997, vol. 16, no. 4, août 1997 (1997-08), pages 307-316, XP008078446 ISSN: 0272-457X cité dans la demande**
• **MEZAZIGH A ET AL: "A MONOCLONAL ANTIBODY REACTING SPECIFICALLY FOR GANGLIOSIDE O-ACETYLATED GD2 IN NEUROECTODERMAL TUMORS" GLYCOCONJUGATE JOURNAL, LUND, SE, vol. 10, no. 4, 20 août 1993 (1993-08-20), pages 300-301, XP008078472 ISSN: 0282-0080**
• **AUBRY JACQUES: "MAb 8B6 anti-O-acetyl GD2 ganglioside" HYBRIDOMA, vol. 16, no. 6, décembre 1997 (1997-12), page 568, XP008078553 ISSN: 0272-457X**

**(Cont. page suivante)**

- YE J N ET AL: "A novel O-acetylated ganglioside detected by anti-GD2 monoclonal antibodies." INTERNATIONAL JOURNAL OF CANCER. JOURNAL INTERNATIONAL DU CANCER 21 JAN 1992, vol. 50, no. 2, 21 janvier 1992 (1992-01-21), pages 197-201, XP002432456 ISSN: 0020-7136
- SJOBERG E R ET AL: "Structural and immunological characterization of O-acetylated GD2. Evidence that GD2 is an acceptor for ganglioside O-acetyltransferase in human melanoma cells." THE JOURNAL OF BIOLOGICAL CHEMISTRY 15 AUG 1992, vol. 267, no. 23, 15 août 1992 (1992-08-15), pages 16200-16211, XP002432457 ISSN: 0021-9258
- HAMILTON W B ET AL: "Ganglioside expression on human malignant melanoma assessed by quantitative immune thin-layer chromatography." INTERNATIONAL JOURNAL OF CANCER. JOURNAL INTERNATIONAL DU CANCER 20 FEB 1993, vol. 53, no. 4, 20 février 1993 (1993-02-20), pages 566-573, XP002432458 ISSN: 0020-7136

- YAN LI ET AL: "Pharmacogenetics and pharmacogenomics in oncology therapeutic antibody development" BIOTECHNIQUES, INFORMA LIFE SCIENCES PUBLISHING, WESTBOROUGH, MA, US, vol. 39, no. 4, octobre 2005 (2005-10), pages 565-568, XP001245630 ISSN: 0736-6205
- AUBRY JACQUES: "MAb 60C3 anti-GD2 ganglioside and its O-acetylated form" HYBRIDOMA, vol. 16, no. 6, décembre 1997 (1997-12), page 566, XP008078467 ISSN: 0272-457X

**Description**

**Domaine de l'invention**

La présente invention vise à fournir de nouveaux moyens pour la thérapie des cancers dont les cellules expriment la forme O-acétylée du ganglioside GD2. La présente divulgation vise aussi à fournir de nouveaux moyens pour le diagnostic des tumeurs dont les cellules expriment la forme O-catalytic du ganglioside GD2. Ces moyens incluent essentiellement l'utilisation d'anticorps monoclonaux recombinants ou des fragments de celui-ci reconnaissant le GD2 O-acétylé -et qui ne reconnaissant pas les cellules saines et les fibres nerveuses du système nerveux périphérique. Ces nouveaux moyens seront plus spécifiques des cellules cancéreuses et réduiront la toxicité des traitements par rapport aux anticorps et dérivés utilisés précédemment pour traiter ces cancers.

**Art antérieur**

Les cellules tumorales possèdent à leur surface un certain nombre de déterminants antigéniques. Parmi ces déterminants antigéniques, certains sont des antigènes spécifiques de la tumeur. Ces antigènes tumoraux sont exprimés principalement voire exclusivement à la surface de cellules cancéreuses.

Ces antigènes tumoraux humains induisent la production, par le système immunitaire d'espèces différentes comme la souris, des molécules appelées anticorps qui ont la particularité de reconnaître précisément les molécules d'antigène à l'origine de leur production.

Cette reconnaissance spécifique entre un anticorps monoclonal et son antigène tumoral humain présente un intérêt majeur pour l'immunociblage des cancers. En effet, elle permet de concentrer au niveau des tissus ou des organes malades, des agents de détection dans un but de diagnostic ou des agents toxiques dans un but de traitement des tumeurs. Ces agents de détection ou toxiques peuvent être des composés chimiques ou biologiques, éventuellement radioactifs, attachés artificiellement aux anticorps avant ou après leur administration. Ces agents peuvent également être des composés biologiques naturellement présents chez le patient (composants du complément, chemokines, cytokines, cellules cytotoxiques, par exemple lymphocytes T ou NK) recrutées vers les cellules tumorales par les anticorps.

Les gangliosides sont des constituants de la membrane cellulaire et certains d'entre eux ont été caractérisés comme étant des antigènes tumoraux. Il a pu être démontré que le ganglioside GD2 est fortement exprimé dans les cancers humains d'origine neuroectodermique tels que notamment les mélanomes, les glioblastomes, les carcinomes pulmonaires à petite cellule et les neuroblastomes. La liste des pathologies tumorales ici citées n'est pas exhaustive. Les rétinoblastomes et les ostéosarcomes expriment également le ganglioside GD2. Certains cancers de l'ovaire expriment également le ganglioside GD2. Le ganglioside GD2 est un glycolipide acide formé par un céramide associé un oligosaccharide dont la séquence est glucose galactose N-acétyl-galactosamine. À la molécule de galactose se trouvent rattachées deux molécules d'acide sialique. L'acide sialique terminal peut être modifié par l'ajout d'un groupement O-acétyl pour former le GD2 O-acétylé. À l'aide d'anticorps spécifiques du GD2 qui reconnaissent également sa forme O-acétylée, il a pu être montré que certains types de cancers, notamment les tumeurs d'origine neuroectodermique expriment le GD2 et sa forme O-acétylée, le GD2 O-acétylé, en proportion variable.

Plusieurs anticorps monoclonaux anti-GD2 produits chez la souris ont été décrits. L'utilisation thérapeutique des anticorps monoclonaux anti-GD2, 14.G2a et 3F8,A novel O-acetylated ganglioside detected by anti-GD2 monoclonal antibodies; J.N.YE et al; Int.J. Cancer, 50, 197-201(1992) appartenant respectivement aux immunoglobulines de classe IgG2a et IgG3 de souris, a été testée dans des études cliniques.

Ces études ont mis en avant plusieurs effets secondaires négatifs inhérents aux anticorps monoclonaux de souris. Un de ces effets négatifs est une réaction allergique ou anaphylactique qui peut être attribuée à une réponse immunitaire développée par l'homme contre les anticorps de souris.

Afin de résoudre ce problème d'immunogénicité des anticorps monoclonaux produits chez la souris, il est possible grâce à des techniques d'ingénierie génétique de réaliser des anticorps chimériques. On appelle anticorps "chimérique" un anticorps modifié génétiquement dans lequel une partie plus ou moins importante de l'information génétique codant l'anticorps de souris par la partie correspondante d'un anticorps humain. Généralement, les régions variables des chaînes lourdes et légères de l'anticorps murins ($V_H$ et $V_L$) sont conservées, tandis que le reste de la molécule d'anticorps provient d'un anticorps humain. Ces anticorps chimériques conservent la reconnaissance spécifique de l'antigène tumoral inhérente à l'anticorps monoclonal de souris tout en présentant les propriétés physiques et effectrices inhérentes à l'anticorps humain. Ainsi, des études cliniques ont démontré que les anticorps chimériques ch.14G2a et ch.14.18 présentent une immunogénicité réduite et une demi-vie sérique accrue en comparaison à l'anticorps monoclonal de souris dont ils dérivent. De plus il a été mis en évidence que les anticorps monoclonaux anti-GD2 de souris présentent une capacité accrue à recruter les cellules effectrices humaines lorsqu'ils sont chimérisés, alors que les anticorps de souris n'ont qu'une capacité limitée, à recruter ces cellules effectrices humaines.

L'identification des anticorps murins aux anticorps humains peut être poussée plus loin en ne conservant de l'anticorps murin que les acides aminés des régions dites CDR ou "complementarity determining regions" qui constituent le site de liaison de l'antigène et déterminent donc la spécificité de l'anticorps. On appelle anticorps "humanisé" un anticorps génétiquement modifié dans lequel on a conservé seulement les acides aminés des CDR, plus éventuellement quelques acides aminés proches de ces régions CDR de l'anticorps de souris de départ, et dans lequel le reste de la molécule provient d'un anticorps humain.

**Inconvénients de l'art antérieur**

Une utilisation thérapeutique ou diagnostique in vivo des anticorps monoclonaux nécessite que ceux-ci réunissent des qualités particulières de spécificité, d'affinité et de non toxicité. Or les études cliniques menées sur les anticorps monoclonaux et chimériques anti-GD2 mettent en avant un effet secondaire négatif majeur, non résolu par la chimérisation ou l'humanisation et qui est la neurotoxicité des anticorps anti-GD2. En effet, les patients présentant un cancer d'origine neuroectodermique auxquels ont été administrés des anticorps anti-GD2 ont présenté des douleurs au cours de l'administration des anticorps et certains de ces patients ont développé une neuropathie du système nerveux périphérique. Ces effets s'expliquent par la présence de gangliosides GD2 à la surface des cellules fibres nerveuses du système périphérique.

On comprend alors que cette toxicité nerveuse inhérente à l'utilisation des anticorps anti-GD2 a limité le développement clinique de l'immunothérapie utilisant ces anticorps. Pourtant le grand nombre de cibles antigéniques GD2 reste un facteur d'intérêt majeur pour l'immunociblage des cancers d'origine neuroectodermique.

Une approche qui permettrait de résoudre le problème de toxicité nerveuse serait d'utiliser un anticorps reconnaissant un antigène tumoral associé aux cancers d'origine neuroectodermique différent du ganglioside GD2 et qui ne reconnaîtrait pas les fibres nerveuses du système périphérique.

Il y quelques années, les inventeurs ont mené une série d'immunisations de souris afin de produire des anticorps anti-GD2. Plusieurs anticorps anti-GD2 ont ainsi pu être isolé et leur caractérisation a conduit notamment à l'identification d'un anticorps de la classe des IgG3 de souris appelé 8B6 reconnaissant spécifiquement une molécule de ganglioside GD2 légèrement modifiée par la présence d'un groupement O-acétyle. Ces travaux, et notamment cet anticorps 8B6 sont décrits dans l'article «Variable Region Gene Segments of Nine Monoclonal Antibodies Specific to Disialogangliosides (GD2,GD3) and their O-Actylated Derivatives », Cerato et al., Hybridoma Volume 16, Number 4, 1997,307-316, ici incorporé par référence.

La structure de cet anticorps a été étudiée, mais son développement n'a pas été poursuivi. En effet, aucun intérêt particulier de ce nouvel anticorps par rapport aux anticorps anti-GD2 connus n'apparaissait alors. Contrairement à la forme non acétylée du GD2, la distribution tissulaire chez l'homme du GD2 O-acétylé reste très peu documentée en raison de l'absence de méthodes de détection sensibles comme les méthodes immunologiques reposant sur l'utilisation d'anticorps monoclonaux spécifique de la forme acétylée du GD2. Il était donc présagé que l'utilisation clinique de ce nouvel anticorps présenterait probablement le même problème de neurotoxicité que l'utilisation des anticorps monoclonaux anti-GD2. De plus aucune activité cytotoxique de l'anticorps 8B6 n'a été mise en évidence et les anticorps IgG3 ne sont pas de manipulation aisée du fait de leur tendance à s'agglutiner

**Objectifs de l'invention**

Les anticorps monoclonaux spécifiques du ganglioside GD2-O-acétylé qui ne reconnaissent pas le ganglioside GD2, pourraient présenter l'avantage de ne pas reconnaître les fibres nerveuses du système périphérique et donc ne pas être à l'origine d'une toxicité nerveuse. De tels anticorps pourraient donc présenter un avantage majeur dans l'immunociblage de certains types de cancers comme les cancers humains d'origine neuroectodermique qui expriment le ganglioside GD2 sous sa forme O-acétylé par rapport aux anticorps monoclonaux anti-GD2.

De tels anticorps pourront être obtenus par une variété de techniques connues de l'homme de l'art. Une partie de ces techniques commencent par l'immunisation d'animaux, notamment de rongeurs et parmi eux les souris, rats ou hamsters, les lagomorphes et notamment les lapins, les camélidés, et parmi eux les lamas. L'immunogène, préparation contenant l'antigène contre lequel on veut obtenir des anticorps, peut être soit le glycolipide lui-même, purifié ou contenu dans un mélange brut ou partiellement purifié, soit le glycolipide ou une de ses parties, notamment la partie glycosylée hydrophile, éventuellement couplé de façon covalente ou non à des protéines ou des lipides servant de "carrier" pour stimuler la réponse immunitaire. Dans un autre procédé d'immunisation, l'immunogène peut être préparé à partir de cellules exprimant l'antigène, obtenues par culture cellulaire de lignées de cellules tumorales ou de cellules primaires obtenues à partir de prélèvements tumoraux. Parmi les lignées utiles on peut citer notamment des lignées de cellules animales, comme le thymome murin EL-4, ou humaines, comme le neuroblastome humain IMR32, le glioblastome humain U87MG, le carcinome pulmonaire à petite cellule HCl-H82 et le mélanome humain M21. - Ces cellules peuvent alors être administrées entières, vivantes ou fixées, ou être fractionnées pour n'injecter par exemple qu'une fraction partiellement purifiée contenant les composants de la membrane cellulaire ou du cytoplasme.

Ces immunogènes pourront être administrés aux animaux par diverses voies, notamment sous-cutanée, intra-péritonéale ou intra-musculaire, seul ou en présence d'un adjuvant, notamment alum ou adjuvant de Freund. Les immunisations peuvent être répétées avec une fréquence pouvant aller de quelques jours à quelques mois.

Trois méthodes générales pourront conduire à l'obtention des anticorps utilisables dans des applications industrielles. La première consiste à prélever le sang des animaux immunisés et à extraire de celui-ci, par des procédés connus de l'homme de l'art, des fractions contenant l'anticorps plus ou moins purifié (sérum ou plasma, immunoglobulines totales). Les anticorps peuvent être encore purifiés par des techniques chromatographiques ou d'immunoadsorption. La deuxième méthode consiste à prélever des cellules capables de synthétiser les anticorps, et notamment des cellules spléniques ou de ganglions lymphatiques, et de les immortaliser, notamment par transformation virale ou par hybridation somatique selon des procédés connus de l'homme de l'art. Parmi les cellules immortalisées, le clonage permet de sélectionner des cellules produisant les anticorps d'intérêt et la culture cellulaire permettra d'isoler à partir des surnageants de culture ces anticorps et de les purifier en grandes quantités. Enfin la troisième méthode consiste à isoler le RNA de cellules capables de synthétiser des anticorps, notamment des cellules spléniques, de ganglions lymphatiques ou des lymphocytes périphériques prélevées à des animaux, immunisés ou non, voire d'êtres humains et de constituer des banques de cDNA à partir desquelles les séquences d'anticorps d'intérêt seront sélectionnées par criblage selon des méthodes connues de l'homme de l'art et notament par expression de cette banque à la surface d'un bactériophage appelé encore système d'expression du *phage display*. La construction de banques combinatoires de régions VH et VL humaines, exprimées à la surface de phages filamenteux ("phage display"), sous forme de fragments « simple chaîne » ("single chain Fv", scFv), liant une région VH et une région VL ou de fragments F(ab), constitués de la chaîne légère associée au segment peptidique VH-CH1, ce dernier correspondant au premier domaine de la région constante.

Les anticorps de la présente invention pourront être sélectionnés à partir des anticorps obtenus par l'une ou l'autre des méthodes décrites ci-dessus en ce qu'ils reconnaissent l'antigène GD2-O-acétylé et pas l'antigène GD2 non O-acétylé, propriété que l'on nommera spécificité requise. Cette sélection pourra être réalisée au cours du procédé d'isolement des anticorps, par exemple en ne propageant que les cellules produisant des anticorps possédant la spécificité requise. Alternativement, on pourra rechercher parmi des anticorps déjà isolés, ceux qui possèdent cette spécificité. À cette fin, une variété de méthodes peuvent être utilisées, notamment l'immunofluorescence indirecte sur cellules pour distinguer ceux des anticorps qui reconnaissent les cellules connues pour exprimer le GD2 O-acétylé, notamment IMR32, de ceux qui reconnaissent également des cellules exprimant le GD2 mais pas le GD2 O-acétylé, notamment Neuro 2A. On pourra également utiliser un test immuno-enzymatique ELISA sur des cellules dessiquées pour sélectionner les anticorps qui se fixent uniquement sur les cellules exprimant le GD2 O-acétylé et pas sur celles qui n'expriment que le GD2. On pourra aussi sélectionner les anticorps présentant la spécificité requise en utilisant la chromatographie couche mince sur silice pour séparer les composants glycolipidiques de cellules exprimant à la fois GD2 O-acétylé et GD2, notamment IMR32, en ne retenant que les anticorps qui marquent seulement la bande correspondant au GD2-O-acétylé. On pourra confirmer ce résultat en détruisant par traitement alcalin le GD2 O-acétylé dans l'extrait lipidique et en vérifiant que le marquage est bien aboli. Des exemples de mise en oeuvre de ces techniques pour la confirmation de la spécificité des anticorps de la présente invention sont donnés dans la description détaillée de l'invention, ci-dessous. D'autres méthodes, visant au même résultat, pourront être mises au point par l'homme de l'art.

Les anticorps de la présente invention pourront être modifiés par une variété de techniques connues de l'homme de l'art pour les adapter à différentes applications. On pourra ainsi utiliser les méthodes connues de l'art antérieur pour produire des anticorps simple chaîne scFv ainsi qu'une variété de protéines de fusion conservant la capacité de lier l'antigène. On sait en particulier que l'on peut modifier entièrement les régions dites constantes des anticorps, par exemple pour échanger des régions constantes murines contre des régions constantes d'anticorps humains, sans perdre la reconnaissance de l'antigène. La construction d'un anticorps chimérique consiste à isoler l'ADN codant pour la région VH et la région VL d'un anticorps monoclonal de souris et à le lier à l'ADN codant les régions constantes H et L d'une immunoglobuline humaine. Une telle construction génétique permet de produire un anticorps hybride dont la partie constante, humaine, n'est pas ou très peu immunogène chez l'Homme (il s'agit en général de la région constante des IgG 1 humaines et de la région Ckappa humaine). On sait même conserver seulement les régions indispensables à la reconnaissance de l'antigène, régions dites hypervariables, régions déterminant la complémentarité ou encore CDR, et remplacer toutes les autres pour opérer ce que l'on appelle une "humanisation" de l'anticorps. L'ensemble de ces protéines sera désigné par le terme « anticorps modifiés artificiellement » pour faciliter la description.

Certaines de ces protéines ont un intérêt particulier pour les anticorps de la présente invention. Par exemple, les anticorps modifiés artificiellement anti-GD2 O-acétylés qui conservent la reconnaissance du ganglioside GD2 O-acétylés sans reconnaître le ganglioside GD2 pourraient présenter un avantage majeur par rapport aux anticorps monoclonaux anti-GD2 O-acétyles de souris en ce qu'ils auraient des caractéristiques physiques et effectrices supérieures.

Les anticorps modifiés artificiellement spécifiques du GD2 O-acétylé devront avoir une affinité suffisante pour l'antigène correspondant de façon à éliminer au maximun la diffusion de cet anticorps, le cas échéant porteur d'une substance toxique ou thérapeutique dans les tissus ou des cellules sains. Dans le cadre de la présente invention, l'affinité pour le ganglioside GD2 devra être supérieure à $10^{-7}$ mole/litre. L'affinité de ces anticorps pourra être augmentée par des techniques connues de l'homme de l'art. Ainsi, l'expression de fragments d'anticorps à la surface de bactériophages constitue un outil précieux pour la recherche de mutants de haute affinité à partir d'un scFv donné. Il est ainsi possible de mimer la maturation d'affinité observée lors du développement de la réponse immunitaire. Les techniques de mutations aléatoires ou de mutations ciblées, suivies d'une sélection par des cycles répétés d'immuno-adsorption/élution ont été ainsi employées avec succès et ont permis d'obtenir des fragments d'anticorps ayant une affinité près de 10 fois supérieure à celle du fragment initial

Un objet particulier de la présente invention, qui ne doit pas limiter celle-ci à cet exemple, concerne les anticorps modifiés artificiellement préparés à partir de l'anticorps 8B6 dont on sait qu'il présente la spécificité requise, ainsi que les protéines recombinantes utilisant les séquences des régions déterminant la complémentarité définies SEQ ID NO : 2 a 4 et SEQ ID NO = 6 à 8

C'est également un objet de l'invention de proposer des modifications des anticorps présentant la spécificité requise afin de leur conférer des propriétés utiles pour le diagnostic ou la thérapeutique de certains cancers. Certaines de ces applications nécessitent l'injection des anticorps ou de leurs dérivés à des malades atteints de certaines pathologies cancéreuses. Dans ce cas, les dérivés dont la séquence se rapproche plus d'une séquence d'anticorps humain seront préférés car ils sont susceptibles de limiter la production par le patient traité d'anticorps contre la molécule injectée, augmentant en cela la tolérance et permettant une administration répétée. De tels dérivés sont par ailleurs susceptibles de favoriser la réponse anticorps contre des déterminants spécifiques particuliers à l'anticorps injecté, dits anticorps anti-idiotypes, qui ont été présentés comme possédant un intérêt thérapeutique.

Pour des applications thérapeutiques, les modifications préférées seront celles qui permettent d'obtenir une activité cytotoxique contre les cellules tumorales exprimant l'antigène cible, ici le GD2O-acétylé.

On sait que seules certaines classes d'immunoglobulines possèdent la propriété d'activer le complément ou d'induire une cytotoxicité à médiation cellulaire. À titre d'exemple, l'anticorps 8B6 reconnaît le GD2O-acétylé mais n'est pas capable d'induire cette cytotoxicité assez efficacement. C'est donc un autre objet de cette invention de proposer des anticorps reconnaissant le GD2 O-acétylé et possédant une activité cytotoxique. Ceci peut être obtenu notamment en remplaçant les régions constantes de l'anticorps par des régions constantes d'anticorps capables d'éliciter cette cytotoxicité et notamment des régions constantes d'immunoglobulines humaines de classe 1. Le pouvoir cytotoxique pourra alors être encore augmenté, notamment en produisant des anticorps glycosylés de façon particulière, et notamment peu fucosylés, par exemple en les faisant produire par des cellules spécialement sélectionnées ou transformées. Des mutations spécifiques, décrites dans la littérature pourront être également introduites dans la séquence de l'anticorps pour le même effet.

Une autre manière de réaliser des anticorps thérapeutiques selon la présente invention est d'associer à un dérivé d'anticorps présentant la spécificité requise un agent cytotoxique. Ledit agent cytotoxique pourra être un agent toxique chimique, des ARN antisens, et notamment un médicament antitumoral cytotoxique, parmi lesquels on peut citer les taxanes, les alcaloïdes de la pervenche et leurs dérivés, les anthracyclines, les agents alkylants, un agent toxique biologique, et notamment les toxines végétales ou bactériennes, parmi les quels on peut citer la ricine ou la toxine de pseudomonas, ou bien encore un isotope radioactif émettant des particules bêta, comme l'iode 131, l'yttrium 90, le lutétium 177, le rhénium 186 ou le cuivre 67, ou des électrons Auger, comme l'indium 111, ou encore des particules alpha, comme le bismuth 213, le bismuth 212 ou l'astate 211, ces exemples ne devant en aucun cas limiter la portée de l'invention. L'anticorps peut être également produit par des méthodes d'ingénierie moléculaire, connues de l'homme de l'art, sous.la forme d'une protéine de fusion associée à une cytokine. Les cytokines utilisées sont essentiellement de la famille des interleukines comme l'interleukine-2 (IL-2), IL-4, IL-5, IL-6, IL-7, IL-10, IL-12, IL-13, IL-14, IL-15, IL-16 et IL-18, des facteurs de croissance hématopéitique tel que le GM-CSF (*granulocyte macrophage Colony Stimulating Factor*) ou le G-CSF (*granulocyte Colony Stimulating Factor*), le *Tumor Necrosis Factor* (TNF), des chemokines. La protéine de fusion anticorps-cytokine obtenue possède les propriétés biologiques de la cytokine et la spécificité de l'anticorps dont elle dérive.

Les anticorps de la présente divulgation pourront également être appliqués avantageusement au diagnostic, que celui-ci soit pratiqué in vitro pour la détection de la présence de l'antigène GD2 O-acétylé dans des prélèvements de cellules ou de fluides biologiques suivant l'une quelconque des techniques connues de l'homme de l'art, ou bien encore pratiqué in vivo par administration d'un dérivé de l'anticorps modifié pour le rendre détectable par une des techniques d'imagerie médicale connues, à savoir scintigraphie ou tomographie d'émission de positons. Dans ce cas, le dérivé de l'anticorps sera associé à un isotope radioactif émetteur de photon gammas, comme l'iode 131, l'iode 123, l'indium 111, ou le technétium 99m pour l'imagerie scintigraphique ou la tomographie de simples photons, ou à un isotope émetteur de positons, comme le fluor 18, l'iode 124, l'yttrium 86, le cuivre 64, le scandium 44, pour la tomographie d'émission de positons, ces exemples ne devant encore une fois pas limiter le champ de l'invention.

Les anticorps de la présente invention pourront être associés à des composés toxiques ou radioactifs. Les produits toxiques sont couplés chimiquement, de façon covalente, aux anticorps par une variété de liaisons chimiques, parmi lesquelles on peut citer les liaisons esters, amides, disulfures ou thiooéthers. Les atomes radioactifs sont couplés soit directement par substitution électrophile (cas des isotopes de l'iode) ou nucléophile (cas du fluor 18) ou par l'intermédiaire d'un synthon radiomarqué réactif, et notamment le réactif de Bolton et Hunter pour les isotopes de l'iode ou les esters activés stannylés pour les isotopes de l'iode ou l'astate 211, ou bien encore à l'aide d'un agent chélatant quand il s'agit d'un métal radioactif. Dans ce dernier cas, l'homme de l'art sait choisir parmi les chélatants celui qui donnera avec le métal un complexe de bonne stabilité dans les fluides biologiques. Ainsi le DTPA pourra être utilisé avantageusement avec l'indium 111, mais le DOTA sera préférable pour un marquage avec l'yttrium 90.

Les anticorps de la présente invention pourront être utilisés dans des méthodes connues de l'homme de l'art dans lesquelles l'agent toxique ou détectable n'est pas lié directement à l'anticorps, mais au contraire lié à une molécule de faible moléculaire administrée dans une deuxième étape, après qu'un dérivé de l'anticorps capable de reconnaître in vivo cette petite molécule a été administré au patient. Dans ce cas le dérivé de l'anticorps est notamment un anticorps bispécifique ou un immunoconjugué ou une protéine de fusion entre un dérivé de l'anticorps et une avidine. Cette approche pourra être utilisée avantageusement avec les anticorps e la présente invention pour le traitement des tumeurs, et également cette approche pourra être utilisée avec les anticorps de la présente divulgation pour le diagnostic in vivo des tumeurs.

L'anticorps de la présente invention et les « dérivés », « anticorps dérivés » ou « produits dérivés » pourront donc être avantageusement utilisés pour thérapeutique de ces tumeurs. L'anticorps de la periode divulgation et les "dérivés", "anticorps dérivés" ou "produits dérivés" pourront donc être utilisés par le diagnostic de ces tumeurs. Parmi celles-ci on trouve les tumeurs d'origine neuroectodermique et notamment les mélanomes, les cancers du poumon à petites cellules, les gliomes et les neuroblastomes. Les produits de la présente invention pourront être appliqués avantageusement à la détection et au traitement de ces tumeurs, notamment lorsque celles-ci sont disséminées ou échappent aux traitements existants.

### Résumé de l'invention

La présente invention décrit l'utilité d'anticorps dans l'immunociblage des cancers humains d'origine neuroectodermique tels que les mélanomes, les glioblastomes, les carcinomes pulmonaires à petite cellule et les neuroblastomes.

Dans l'ensemble des différents aspects de l'invention décrit ci-dessus, il est particulièrement surprenant de constater qu'un anticorps monoclonal spécifique du GD2 O-acétylé ne se fixe pas sur les fibres nerveuses exprimant le GD2 alors qu'il reconnaissait les cellules tumorales exprimant le ganglioside GD2 et sa forme O-acétylée. De tels anticorps possèdent donc une spécificité restreinte aux cellules tumorales d'origine neuroectodermique, et ne reconnaissant pas les fibres nerveuses du système périphérique. La conséquence de cette spécificité accrue est une toxicité réduite dans les applications thérapeutiques résultant notamment de l'absence de fixation sur les tissus nerveux périphériques normaux qui est observée avec les anticorps reconnaissant le ganglioside GD2. La présente invention couvre donc l'utilisation de tels anticorps pour le diagnostic et la thérapeutique des cancers avec une spécificité accrue et une toxicité réduite par rapport aux anticorps qui reconnaissent le GD2.

Les anticorps de la présente invention ont une activité cytotoxique pour les cellules tumorales qu'ils reconnaissent, soit intrinsèquement (c'est une des raisons pour lesquelles on les chimérise ou humanise), soit parce qu'ils servent de vecteurs pour des agents toxiques, radioactifs notamment.

La présente divulgation couvre donc tout anticorps monoclonal chimérique ou humanisé ne reconnaissant que la forme O-acétylée du ganglioside GD2, ou fragment de cet anticorps, ledit anticorps ou ledit fragment reconnaissant les molécules de GD2 O-acétylé exprimées par des cellules tumorales et ne reconnaissant pas les molécules de GD2 exprimées à la surface des nerfs périphériques.

La présente divulgation couvre également les anticorps dont certains amino-acides ont été remplacés par d'autres en utilisant les techniques de génétique moléculaire connues de l'homme de l'art, notamment pour modifier les propriétés de l'anticorps original, en particulier pour diminuer son immunogénicité, ou pour augmenter son activité toxique ou encore pour accélérer ou ralentir son elimination après injection.

Avantageusemet l'anticorps monoclonal modifié artificiellement ou fragment de celui-ci selon la divulgation est caractérisé en ce qu'il est une IgG kappa d'affinité supérieure à $10^{-7}$ mole/litre pour le GD2-O-acétylé et d'affinité au moins dix fois plus faible pour le GD2 lui-même, ledit anticorps ou ledit fragment étant mono ou bispécifique.

Notamment, mais non exclusivement, la divulgation couvre tout anticorps monoclonal modifié artificiellement ou fragment de celui-ci où les régions déterminant la complémentarité de la région variable de la chaîne H ont comme séquences d'aminoacides celles représentées dans SEQ ID NO:2. SEQ ID NO:3 et SEQ ID NO:4 et les régions déterminant la complémentarité de la région variable de la chaîne L ont comme séquences d'aminoacides celles représentées dans SEQ ID NO:6, SEQ ID NO:7 et SEQ ID NO:8.

Notamment, mais non exclusivement, la divulgation couvre tout anticorps monoclonal modifié artificiellement ou fragment de celui-ci, dont la chaîne lourde est obtenue par la jonction entre l'ADNc codant la région variable de la chaîne lourde d'un anticorps non humain et l'ADNc codant la région constante d'une immunoglobuline humaine et dont la chaîne légère est obtenue par la jonction entre l'ADNc codant la région variable de la chaîne légère du même anticorps non humain et l'ADNc codant la région constante de la chaîne légère d'une Immunoglobuline humaine caractérisé en ce que ledit anticorps non humain est l'anticorps monoclonal de souris 8B6 et ledit anticorps modifié artificiellement est dirigé contre le ganglioside GD2 O-acétylé et ne reconnaît pas les fibres nerveuses du système périphérique.

Notamment, mais non exclusivement, la divulgation couvre tout anticorps monoclonal modifié artificiellement ou fragment de celui-ci dont la région variable de la chaîne lourde possède la séquence déduite en acides aminés définie par SEQ ID NO:1 et dont la région variable de la chaîne légère possède la séquence déduite en acides aminés définie par SEQ ID NO :5

Préférentiellement, l'anticorps monoclonal modifié artificiellement ou fragment de celui-ci selon la revendication 5, est l'anticorps KM8B6 pouvant être obtenu à l'aide de la lignée cellulaire CHO ou un fragment de celui-ci.

La divulgation couvre également toute molécule pharmaceutique dérivée de l'anticorps modifié artificiellement ou d'un fragment de celui-ci selon l'invention dans laquelle l'anticorps ou le fragment de celui-ci est couplé avec une molécule X, où X est une molécule toxique, un médicament, une pro-drogue, ou un deuxième anticorps quelle que soit sa spécificité.

Selon une variante, ladite molécule toxique est une molécule toxique chimique, biologique ou radioactive, ladite molécule étant destinée à détruire des cellules tumorales exprimant le ganglioside GD2-O-acétylé.

Préférentiellement lesdites cellules tumorales visées par les molécules pharmaceutiques selon l'invention sont des cellules de neuroblastome, de mélanome, de glioblastome ou de cancer pulmonaire à petites cellules.

Préférentiellement les molécules pharmaceutiques selon la divulgation sont mutées au niveau de leur région Fc par l'adjonction de sucres, modulant ainsi l'activation des cellules immunitaires et des molécules du système du complément.

La divulgation couvre également toute molécule pour le diagnostic des cancers montrant une expression du ganglioside GD2 O-acétylé à la surface des cellules tumorales, ladite molécule étant dérivée de l'anticorps modifié artificiellement ou d'un fragment de celui-ci selon l'invention, dans laquelle ledit anticorps ou ledit fragment est lié à un agent permettant la détection de l'anticorps ou dudit fragment par la fluorescence ou la radioactivité.

La divulgation concerne donc toute utilisation d'un anticorps modifié artificiellement ou d'un fragment de celui-ci selon l'invention et/ou d'une molécule selon l'invention, pour la fabrication d'un médicament pour la thérapie d'un cancer dont les cellules expriment la forme O-acétylée du ganglioside GD2 ou pour la fabrication d'un produit pour le diagnostic d'un tel cancer.

La présente invention couvre également toute utilisation de l'anticorps monoclonal 8B6 (décrit dans l'article « Variable Region Gene Segments of Nine Monoclonal Antibodies Specific to Disialogangliosides (GD2, GD3) and their O-Actylated Derivatives », Cerato et al., Hybridoma Volume 16, Number 4, 1997,307-316) pour la fabrication d'une molécule pharmaceutique dans laquelle ledit anticorps est lié à un agent toxique chimique, biologique ou radioactif, ladite molécule étant destinée à détruire des cellules tumorales exprimant le ganglioside GD2-O-acétylé.

Notamment, mais non exclusivement, l'invention couvre une telle utilisation lorsque lesdites cellules sont des cellules de neuroblastome, de mélanome, de glioblastome ou de cancer pulmonaire à petites cellules.

Notamment, mais non exclusivement, l'invention couvre une telle utilisation lorsque ladite molécule thérapeutique est mutée au niveau de sa région Fc par l'adjonction de sucres, modulant ainsi l'activation des cellules immunitaires et des molécules du système du complément.

La divulgation couvre également toute utilisation de l'anticorps monoclonal 8B6 pour la fabrication d'une molécule pour le diagnostic des cancers montrant une expression du ganglioside GD2-O-acétylé à la surface des cellules tumorales, ladite molécule étant dérivée dudit anticorps, dans laquelle ledit anticorps est lié à un agent permettant la détection de l'anticorps par la fluorescence ou la radioactivité.

La divulgation couvre aussi toute séquence d'ADN codant pour l'anticorps modifié artificiellement selon l'invention ainsi que tout vecteur d'expression comprenant telle séquence d'ADN liée de façon opérationnelle à un promoteur.

La divulgation couvre aussi toute cellule, notamment animale, comprenant un tel vecteur d'expression, ainsi que tout transformant non humain qui produit l'anticorps modifié artificiellement selon l'invention.

Enfin la divulgation couvre tout procédé pour la production d'anticorps modifié artificiellement pour le ganglioside GD2-O-acétylé, ce procédé comprenant l'expression de la séquence d'ADN dans une cellule ou un transformant non humain dans des conditions appropriées et la récupération de l'anticorps.

Préférentiellement, la cellule ou le transformant est cultivé dans des conditions dans lesquelles l'anticorps s'accumule.

**Brève description des figures**

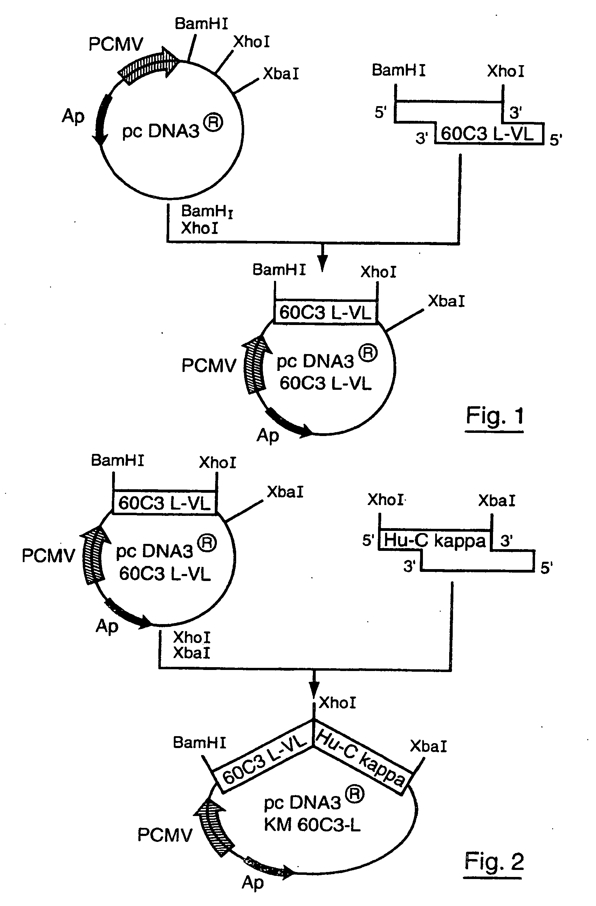

    Fig. 1 décrit la construction du plasmide, pcDNA3®60C3 L-VL.
    Fig. 2 décrit la construction du plasmide, pcDNA3®60C3 L.

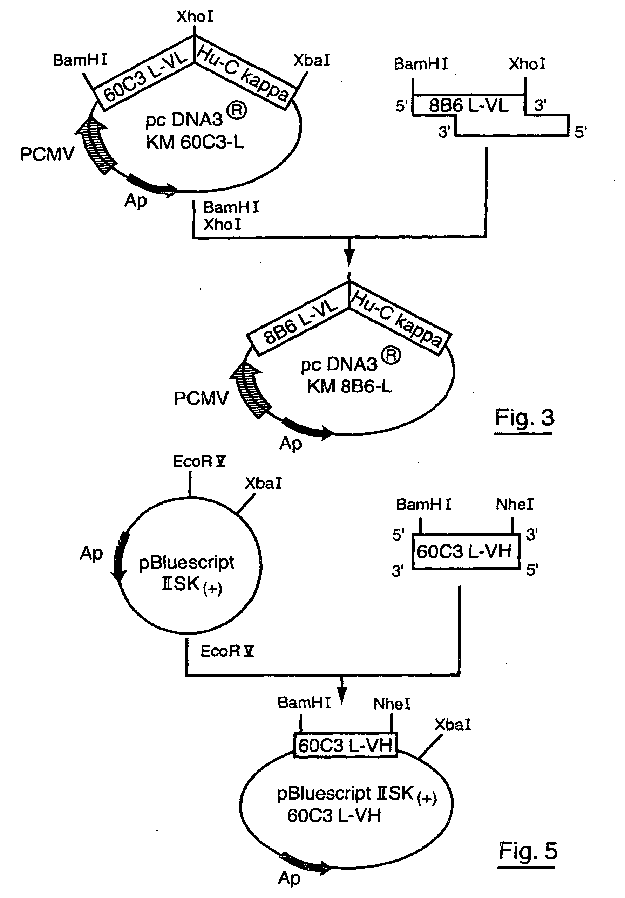

    Fig. 3 décrit la construction du plasmide, pcDNA3® KM8B6L.

Fig.4 décrit la séquence nucléotidique et la séquence déduite en acides aminés de la chaîne légère de l'anticorps modifié artificiellement KM8B6.

Fig. 5 décrit la construction du plasmide, pBluescript® II SK (+) 60C3 L-VH.

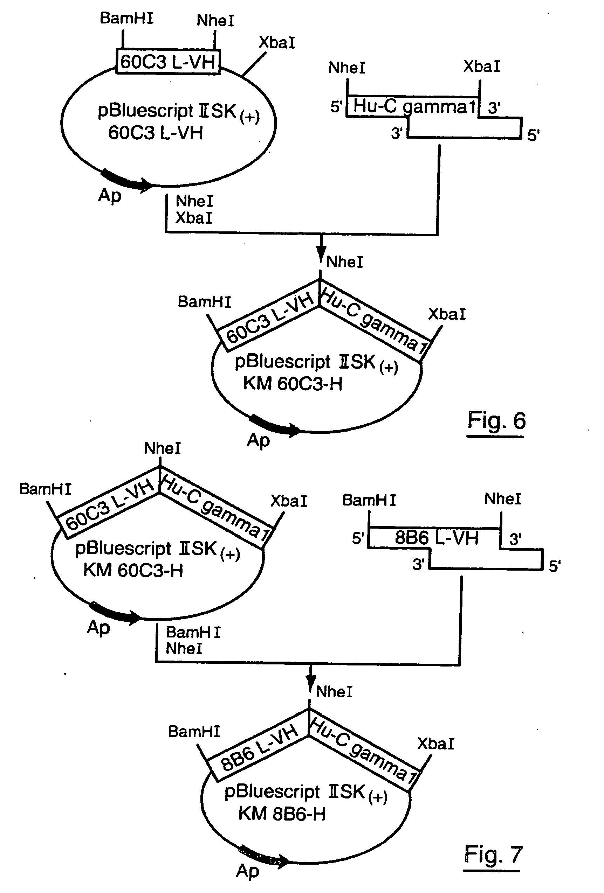

Fig. 6 décrit la construction du plasmide, pBluescript® II SK (+) KM60C3-H.

Fig. 7 décrit la construction du plasmide, pBluescript® II SK (+) KM8B6-H.

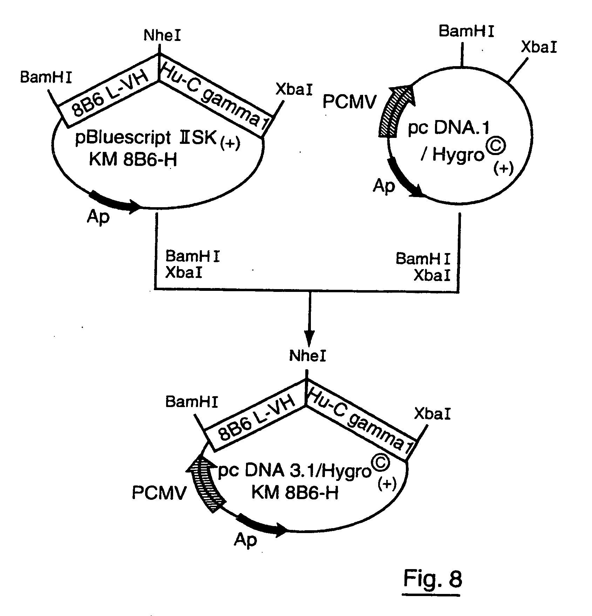

Fig. 8 décrit la construction du plasmide, pcDNA3.1/Hygro© (+)KM8B6-H.

Fig. 9 décrit la séquence nucléotidique et la séquence déduite en acides aminés de la chaîne lourde de l'anticorps modifié artificiellement KM8B6.

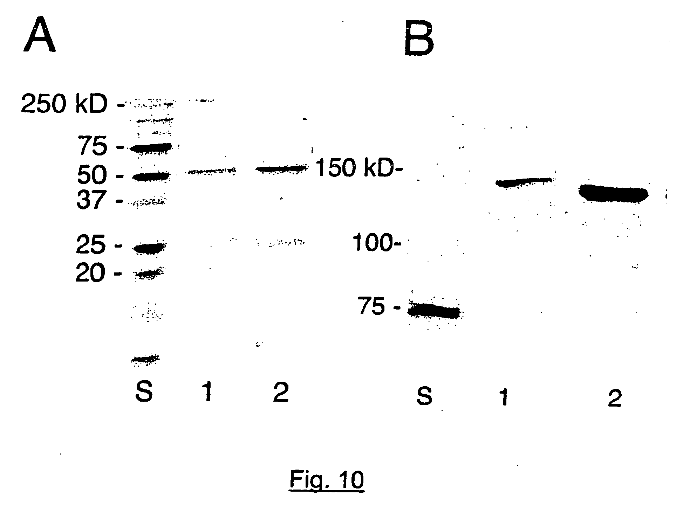

Fig. 10 décrit l'analyse SDS-PAGE de l'anticorps modifié artificiellement anti-GD2-O-acétylé purifié, KM8B6. L'analyse a été réalisée dans des conditions réductrices (gauche) et non réductrices (droite). De gauche à droite, marqueur de poids moléculaire, KM8B6, IgG3 8B6 (conditions réductrices), marqueur de haut poids moléculaire, KM8B6, and IgG3 8B6 (conditions non réductrices).

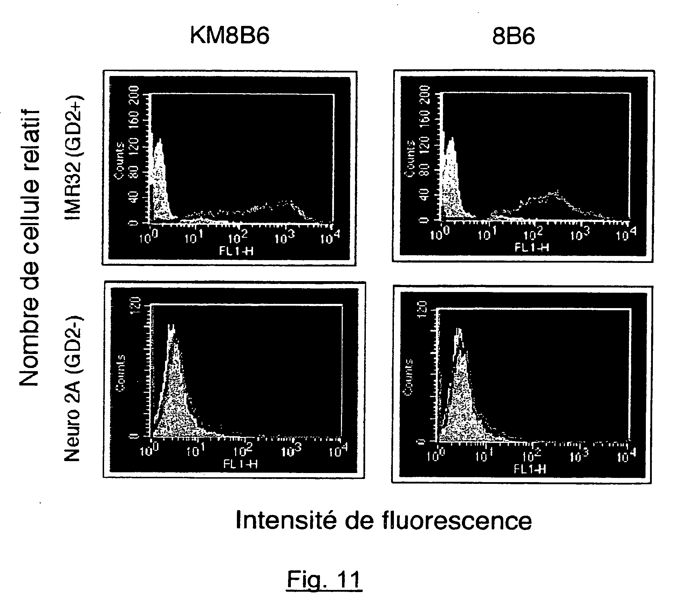

Fig. 11 est un graphique qui décrit la réactivité de l'anticorps 8B6 et de l'anticorps KM8B6 sur des cellules IMR 32 and NeuroA, possédant ou non les antigènes GD2-O-acétylés respectivement, mesurée par immunofluorescence, avec en ordonnées le nombre de cellules détectées et en abscisse l'intensité de fluorescence. Le tracé bleu correspond à la réactivité du contrôle et le tracé rouge correspond à la réactivité des produits.

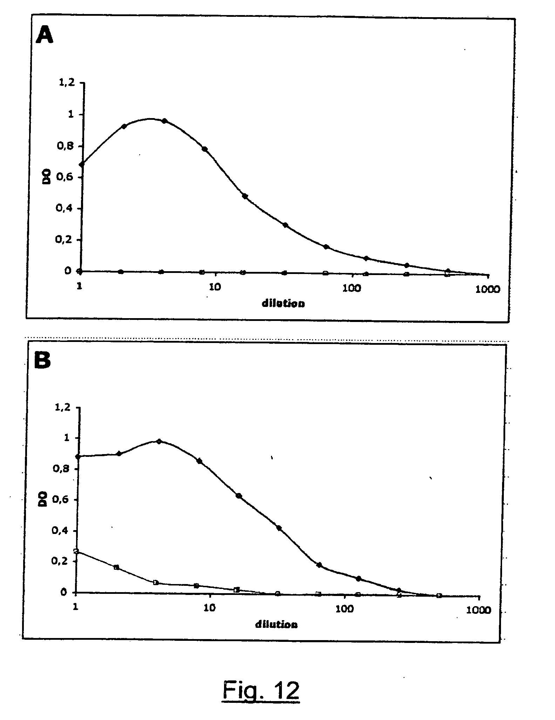

Fig. 12 est un graphique qui décrit la réactivité des anticorps 8B6 et KM8B6 analysée par test ELISA sur des cellules IMR 32 et NeuroA.

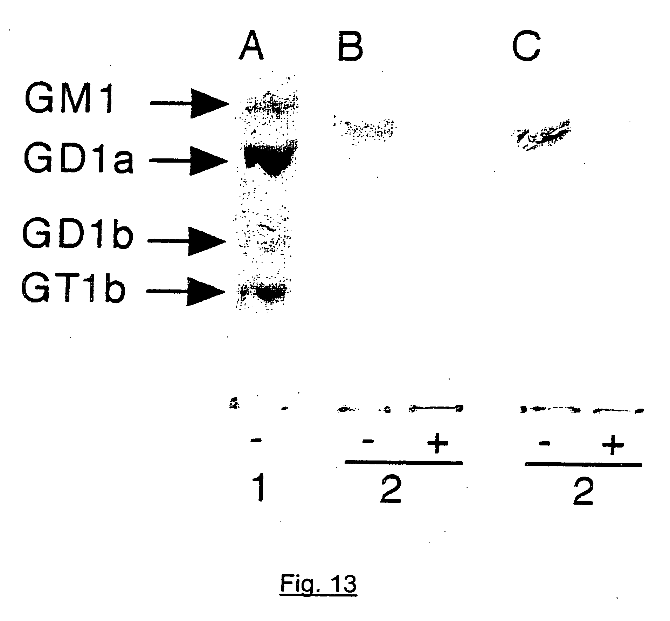

Fig. 13 montre le profil d'immunocoloration obtenu avec l'anticorps KM8B6 de gangliosides de cerveau de rat séparés par chromatographie sur couche mince de silice. Lane A coloration au résorcinol des migrations des différents gangliosides de cerveau de rat.

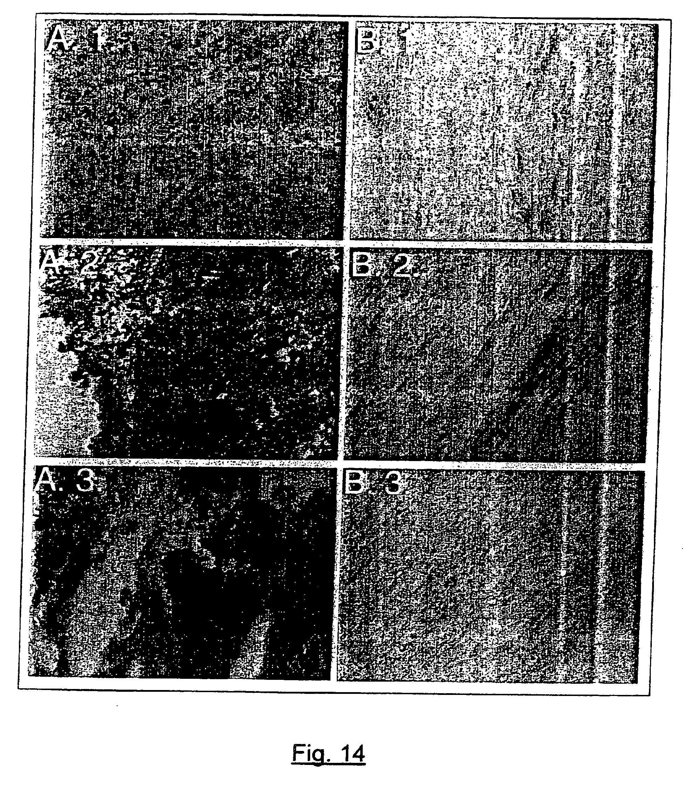

Fig. 14 montre les résultats de l'analyse immuno-histochimique de cellules de neuroblatome et de fibres nerveuses humains.

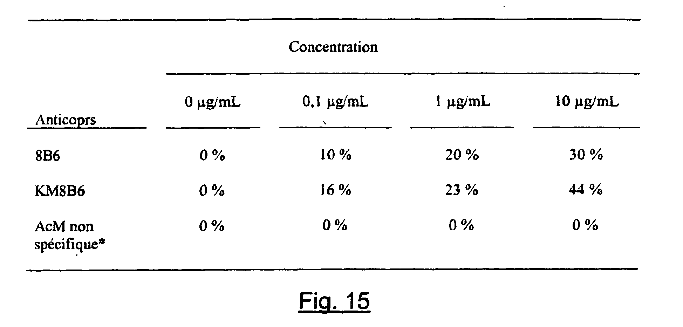

Fig. 15 montre les résultats de l'étude de toxicité (ADCC) Pourcentage d'activité ADCC de l'anticoprs modifié artificiellement KM8B6 et de l'AcM de souris 8B6 dont il dérive. * Anticorps Rituxan® anti-CD20 utilisé comme contrôle négatif

Fig. 16 montre les résultats de l'étude in vivo chez la souris de l'effet anti-tumoral de l'AcM 8B6 contre le thymome murin EL4 qui exprime l'antigène GD2-O-acétylé.

## Description détaillée de l'invention

Production d'anticorps modifié artificiellement KM8B6

1. Préparation de l'ADNc codant la région variable L-VL de l'anticorps monoclonal de souris 60C3

(a). Extraction de l'ARN total de l'hybridome 60C3, producteur de l'anticorps monoclonal 60C3.

L'ARN total est extrait à partir de 106 cellules d'hybridome 60C3 en phase exponentielle de croissance grâce au réactif RNAble (Eurobio, Courtaboeuf, France) selon les recommandations du fournisseur. La concentration en ARN total est déterminée par mesure de la densité optique à 260 nm.

(b). Obtention de la séquence nucléotidique de l'ADNc 60C3 L-VL

L'amplification génique de l'ADNc codant la région variable 60C3 L-VL a été obtenue à partir des ARN messagers par RACE-PCR de façon à obtenir la séquence nucléotidique codant le peptide signal (L) associé à sa région variable (VL). Cette amplification a été réalisée en utilisant le coffret SMART™ RACE cDNA Amplification obtenu auprès de la société BD Biosciences (San José, CA, USA) selon les recommandations du fournisseur. La quantité d'ARN total utilisé pour la rétrotranscription est de 1µg. Le produit de la réaction a été dilué dans 100µL de solution tampon tricine EDTA fourni par le fournisseur. Un volume de 2,5µL du produit dilué a été utilisé pour l'amplification génique. L'amorce antisens spécifique de l'ADNc 60C3 VL utilisée est la suivante : 3'- 60C3 VL 5'- TTT CAG CTC CAG CTT GGT CCC AGC -3'. L'amplification a été réalisée par incubation du mélange réactionnel dans un thermocycleur Perkin-Elmer (PE) DNA thermal Cycler 480 (Perkin Elmer Wellesley, MA, USA) dans les conditions suivantes : 5 cycles (94°C pendant 5 secondes, 72°C pendant 3 minutes), suivi de 5 cycles (94°C pendant 5 secondes puis 70°C pendant 10 secondes suivi de 3 minutes à 72°C) et de 25 cycles (94°C pendant 5 secondes suivi de 69°C pendant 10 secondes et 3 minutes à 72°C). Le produit de réaction de RACE-PCR est analysé par électrophorèse sur gel d'agarose 1% (Q. Biogene, Morgan Irvine, CA, USA) dans une solution tampon de migration de Tris EDTA pH 8 (40 mM Tris-base (Sigma Chemicals Co), 25 mM EDTA (Inerchim, Montluçon, France), 20 mM acide acétique (Carlo Erba Reagenti SpA, Rodano, MI, Italie). Les produits de poids moléculaire attendu sont ensuite purifiés à l'aide du coffret QIAquick Gel Extraction (Qiagen) selon les recommandations du fournisseur. La détermination de la séquence en acide nucléique des produits obtenus purifiés a été réalisée par la société GENOME express (Meylan, France) afin de vérifier la séquence de l'ADNc codant la région 60C3 L-VL. La séquence correspondant au peptide signal associé à la région variable de la chaîne légère de l'anticorps 60C3 a été ainsi déterminée et des amorces dessinées pour permettre le clonage de l'ADNc 60C3 L-VL dans le vecteur d'expression.

(c). Amplification de l'ADNc 60C3 L-VL

L'amplification de l'ADNc 60C3 L-VL a été obtenue par réaction de RT-PCR à partir d'un extrait d'ARN total de cellules d'hybridome 60C3. Le mélange réactionnel est le suivant : Oligo d(T)18 0,5µg (New England Biolabs Inc. Beverly, MA, USA), ARN 1 µg, dNTP 0,5 mM (Promega, Madison, WI, USA), eau stérile qsp 12µL. Ce mélange est incubé 5 minutes à 65°C (bain marie sec) puis 2 minutes à 4°C (glace fondante) pour dénaturer l'ARN. Sont ensuite ajoutés au mélange réactionnel, 4µL de solution tampon 5X First-strand Buffer (Invitrogen Life Biotechnologies), 10 mM DTT (Invitrogen Life Biotechnologies), 160 U de Rnasine (Promega) et 800 U de transcriptase inverse (Invitrogen Life Biotechnologies). L'ensemble est incubé 1 heure à 37°C puis à 70°C pendant 15 minutes afin d'arrêter la réaction. Les copies d'ADNc 60C3 L-VL sont obtenues par amplification génique grâce à une réaction de PCR utilisant les oligonucléotides de synthèse sont les suivantes : 5'- BamHI 60C3 L-VL : 5'- ag gga tcc aaa gac aaa atg gat -3' (amorce sens) et 3'- XhoI 60C3 L-VL : 5'- tt cag ctc gag ctt ggt ccc agc acc -3' (amorce anti-sens)

Les amorces de synthèse utilisées permettent l'introduction de mutations silencieuses dans la séquence d'ADN qui n'entraînent aucune modification au niveau de la séquence en acides aminés tout en permettant l'apparition des sites de restriction BamHI et XhoI nécessaires au clonage dans le vecteur.

Le milieu réactionnel a la composition suivante : dNTP (Promega) 500 µM, Taq polymerase (Invitrogen Life Technologies) 1µL, solution tampon de PCR (Invitrogen Life Technologies) 10µL, ADNc (matrice) 1µL, MgCl2 (Invitrogen Life Technologies) 1,5 mM, amorce 3'- XhoI 60C3 L-VL et amorce 5'- BamHI 60C3 500µM.

L'amplification a été réalisée dans un thermocycleur MJ-research PTC 200 (Peltier Thermal Cycler) selon les conditions suivantes : 5 minutes à 94°C, puis 35 cycles suivants 30 secondes à 94°C, 45 secondes à 55°C et 1 minute à 72°C.

Après vérification des produits de PCR par analyse électrophorétique sur gel d'agarose à 1 %, le cDNA 60C3 L-VL est purifié à l'aide du coffret QIAquick Gel Extraction (Qiagen) selon les recommandations du fournisseur.

2. Construction d'un plasmide recombinant possédant l'ADNc 60C3 L-VL : pcDNA3® 60C3 L-VL

(a). Digestion du vecteur pcDNA3® et de l'ADNc 60C3 L-VL et déphosphorylation du vecteur digéré

La digestion par les enzymes de restriction permet d'obtenir un vecteur linéaire présentant des extrémités cohésives nécessaires pour l'insertion de l'ADNc d'intérêt. La digestion se fait dans les conditions suivantes : tampon NEB2 10X (5 µL), BSA 100X (0,5 µL), vecteur pcDNA33® ou cDNA 60C3 L-VL (1 µg), enzyme de restriction BamHI (10U), enzyme de restriction XhoI (10U). Les enzymes de restriction, les solutions tampons de réaction et la solution de BSA sont de New England Biolabs Inc. La réaction est poursuivie 3 heures au bain marie à 37°C. Le vecteur et l'insert digérés sont purifiés grâce au coffret QIAquick Gel Extraction (Qiagen) selon les recommandations du fournisseur. Le vecteur est ensuite déphosphorylé par la CIP (New Englan Biolabs Inc.) pendant 1 heure à 37°C. La composition du mélange réactionnel est la suivante : tampon NEB3 10X (5 µL), vecteur (1 µg), CIP (1µL). Cette réaction permet d'augmenter le rendement de la ligature en diminuant le pourcentage d'autoligation du vecteur. En effet, le fait de supprimer les groupements phosphate aux extrémités 5' et 3' des bords libérés par la digestion empêche la fermeture spontanée du vecteur.

(b). Réaction de ligation

La ligation permet d'insérer l'ADNc 60C3 L-VL digéré dans le vecteur pcDNA3@ digéré et déphosphorylé grâce à la T4 ligase (New England Biolabs Inc.). La réaction est poursuivie au bain marie à 16°C pendant 16 heures dans le millieu réactionnel suivant : solution tampon de ligature 5X (4 µL) (New England Biolabs Inc.), vecteur digéré déphosphorylé 200 ng, T4 ligase (400U) (New England Biolabs Inc.), ADNc purifié 170 ng (pour un insert de 1 kb). Le rapport molaire insert/vecteur est de 3/1.

(c). Transformation de bactéries compétentes E. coli XL1 blue (Stratagene)

20µL de produit de la réaction de ligation est ajouté à 100µL de suspension de bactéries compétentes. L'ensemble est incubé à 4°C pendant 30 minutes. Les bactéries sont alors soumises à un choc thermique de 2 minutes à 42°C, suivies de 2 minutes à 4°C. Un volume de 1 mL de milieu LB est alors ajouté et les bactéries sont incubées 1 heure à 37°C sous agitation (250 rpm). Les bactéries sont récupérées par centrifugation pendant 5 minutes à 4000 g à 4°C. Le culot bactérien est repris dans 100$\mu$L de milieu LB avant d'être étalé sur une boîte de Pétri contenant le milieu 2XTY agar contenant 100$\mu$g/mL d'ampicilline (Sigma Chemicals Co) et 12,5$\mu$g/mL de tétracycline (Sigma Chemicals Co). Les colonies résistantes apparaissent après une nuit d'incubation à 37°C.

(d). Vérification de la présence de l'insert 60C3 L-VL dans le vecteur pcDNA3® par réaction de PCR

Des colonies isolées sont déposées stérilement sous hotte respectivement dans un tube contenant le mélange réactionnel de PCR : dNTP (Promega) 500 $\mu$M, Taq polymerase (Invitrogen Life Technologies) $\mu$L, solution tampon de PCR (Invitrogen Life Technologies) 10$\mu$L, ADNc (matrice) 1$\mu$L, MgCl2 (Invitrogen Life Technologies) 1,5 mM, amorce 3'- XhoI 60C3 L-VL et amorce 5'- BamHI 60C3 500$\mu$M.

L'amplification a été réalisée dans un thermocycleur MJ-research PTC 200 selon les conditions suivantes : 5 minutes à 94°C, puis 35 cycles suivants 30 secondes à 94°C, 45 secondes à 55°C et 1 minute à 72°C. La taille du produit PCR amplifié est contrôlée par analyse électrophorétique sur gel d'agarose 1 %. Les clones qui ont été correctement transformés sont les seuls à donner un produit de PCR de taille attendue.

(e). Production du plasmide d'intérêt pcDNA3® 60C3 L-VL

On utilise un cure-dent stérile qui après prélèvement de la colonie est mis dans 5 mL de milieu liquide sélectif LB contenant 100$\mu$g/mL d'ampicilline (Sigma Chemicals Co) et 12,5$\mu$g/mL de tétracycline (Sigma Chemicals Co) et incubé 16 heures à 37°C. La dilution bactérienne est alors utilisée pour la réalisation d'une minipréparation d'ADN plasmidique en utilisant le coffret QIAprep spin miniprep (QIAGEN) selon les recommandations du fournisseur.

3. Préparation de l'ADNc codant la région constante hu-C kappa d'un anticorps monoclonal humain P3Non2

(a). Extraction de l'ARN total de l'hybridome P3Non2, producteur de l'anticorps monoclonal P3Non2.

L'ARN total est extrait à partir de 106 cellules d'hybridome P3Non2 en phase exponentielle de croissance grâce au réactif RNAble (Eurobio, Courtaboeuf, France) selon les recommandations du fournisseur. La concentration en ARN total est déterminée par mesure de la densité optique à 260 nm.

(b). Amplification de l'ADNc hu-C kappa

L'amplification de l'ADNc hu-C kappa a été obtenue par réaction de RT-PCR à partir d'un extrait d'ARN total de cellules d'hybridome P3Non2. Le mélange réactionnel est le suivant : Oligo d(T)18 0,5$\mu$g (New England Biolabs Inc. Beverly, MA, USA), ARN 1$\mu$g, dNTP 0,5 mM (Promega, Madison, WI, USA), eau stérile qsp 12$\mu$L. Ce mélange est incubé 5 minutes à 65°C (bain marie sec) puis 2 minutes à 4°C (glace fondante) pour dénaturer l'ARN. Sont ensuite ajoutés au mélange réactionnel, 4$\mu$L de solution tampon 5X First-strand Buffer (Invitrogen Life Biotechnologies), 10 mM DTT (Invitrogen Life Biotechnologies), 160 U de Rnasine (Promega) et 800 U de transcriptase inverse (Invitrogen Life Biotechnologies). L'ensemble est incubé 1 heure à 37°C puis à 70°C pendant 15 minutes afin d'arrêter la réaction. Les copies d'ADNc 60C3 L-VL sont obtenues par amplification génique grâce à une réaction de PCR utilisant les oligonucléotides de synthèse sont les suivantes : 5'- XhoI hu-C kappa: 5'- ag ctc gag ctg aaa cga act gtg gct gca c -3' (amorce sens) et 3'-XbaI hu-C kappa : 5'- ctt cta gat tta aca ctc tcc cct gtt ga -3' (amorce anti-sens).

Les amorces de synthèse utilisées permettent l'introduction de mutations silencieuses dans la séquence d'ADN qui n'entraînent aucune modification au niveau de la séquence en acides aminés tout en permettant l'apparition des sites de restriction XbaI et XhoI nécessaires au clonage dans le vecteur.

Le milieu réactionnel a la composition suivante : dNTP (Promega) 500 $\mu$M, Taq polymerase (Invitrogen Life Technologies) 1$\mu$L, solution tampon de PCR (Invitrogen Life Technologies) 10$\mu$L, ADNc (matrice) 1$\mu$L, MgCl2 (Invitrogen Life Technologies) 1,5 mM, amorce 3'-XbaI hu-C kappa et amorce 5'- XhoI hu-C kappa 500$\mu$M.

L'amplification a été réalisée dans un thermocycleur MJ-research PTC 200 (Peltier Thermal Cycler) selon les conditions suivantes : 5 minutes à 94°C, puis 35 cycles suivants 30 secondes à 94°C, 45 secondes à 55°C et 1 minute à 72°C.

Après vérification des produits de PCR par analyse électrophorétique sur gel d'agarose à 1%, le cDNA hu-C kappa est purifié à l'aide du coffret QIAquick Gel Extraction (Qiagen) selon les recommandations du fournisseur.

4. Construction du vecteur d'expression de la chaîne légère de l'anticorps modifié artificiellement KM 60C3 : pcDNA3® KM60C3-L

(a). Digestion du vecteur pcDNA3® 60C3 L-VL et de l'ADNc hu-C kappa et déphosphorylation du vecteur digéré

La digestion se fait dans les conditions suivantes : tampon NEB2 10X (5 μL), BSA 100X (0,5 μL), vecteur pcDNA3® 60C3 L-VL ou ADNc hu-C kappa (1 μg), enzyme de restriction XhoI (10U), enzyme de restriction XbaI (10U). Les enzymes de restriction, les solutions tampons de réaction et la solution de BSA sont de New England Biolabs Inc. La réaction est poursuivie 3 heures au bain marie à 37°C. Le vecteur et l'insert digérés sont purifiés grâce au coffret QIAquick Gel Extraction (Qiagen) selon les recommandations du fournisseur. Le vecteur est ensuite déphosphorylé par la CIP (New Englan Biolabs Inc.) pendant 1 heure à 37°c. La composition du milieu réactionnel est la suivante : tampon NEB3 10X (5 μL), vecteur (1 μg), CIP (1μL).

(b). Réaction de ligation

La ligation permet d'insérer l'ADNc ADNc hu-C kappa digéré dans le vecteur pcDNA3® 60C3 L-VL digéré et déphosphorylé grâce à la T4 ligase (New England Biolabs Inc.). La réaction est poursuivie au bain marie à 16°C pendant 16 heures dans le millieu réactionnel suivant : solution tampon de ligature 5X (4 μL) (New England Biolabs Inc.), vecteur digéré déphosphorylé 200 ng, T4 ligase (400U) (New England Biolabs Inc.), ADNc purifié 170 ng (pour un insert de 1 kb). Le rapport molaire insert/vecteur est de 3/1. Le produit de réaction de ligature est utilisé pour la transformation de bactéries compétentes E. coli XL1 blue (Stratagene).

(c). Vérification de la présence de l'insert hu-Ckappa dans le vecteur pcDNA3® 60C3 L-VL par réaction de PCR

Les colonies résistantes isolées sont déposées stérilement sous hotte dans un tube contenant le mélange réactionnel de PCR : dNTP (Promega) 500 μM, Taq polymerase (Invitrogen Life Technologies) 1μL, solution tampon de PCR (Invitrogen Life Technologies) 10μL, ADNc (matrice) 1μL, MgCl2 (Invitrogen Life Technologies) 1,5 mM, amorce 3'-XbaI hu-C kappa et amorce 5'- XhoI hu-C kappa 500μM. L'amplification a été réalisée dans un thermocycleur MJ-research PTC 200 selon les conditions suivantes : 5 minutes à 94°C, puis 35 cycles suivants 30 secondes à 94°C, 45 secondes à 55°C et 1 minute à 72°C. La taille du produit PCR amplifié est contrôlée par analyse électrophorétique sur gel d'agarose 1 %. Les clones qui ont été correctement transformés sont les seuls à donner un produit de PCR de taille attendue.

(d). Production du plasmide d'intérêt pcDNA3® KM60C3-L

On utilise un cure-dent stérile qui après prélèvement de la colonie est mis dans 5 mL de milieu liquide sélectif LB contenant 100μg/mL d'ampicilline (Sigma Chemicals Co) et 12,5μg/mL de tétracycline (Sigma Chemicals Co) et incubé 16 heures à 37°C. La dilution bactérienne est alors utilisée pour la réalisation d'une minipréparation d'ADN plasmidique en utilisant le coffret QIAprep spin miniprep (QIAGEN) selon les recommandations du fournisseur.

5. Construction du vecteur d'expression de la chaîne légère de l'anticorps modifié artificiellement KM 8B6 : pcDNA3® 8B6-L

(a). Obtention du vecteur pcDNA3® hu-C kappa

Le vecteur pcDNA3® hu-C kappa a été préparé à partir du vecteur pcDNA3® KM60C3-L par digestion utilisant les enzymes de restriction BamHI et XhoI. La composition du milieu réactionnel est la suivante : tampon NEB2 10X (5 μL), BSA 100X (0,5 μL), vecteur (1 μg), enzyme de restriction BamHI (10U), enzyme de restriction XhoI (10U). Les enzymes de restriction, les solutions tampons de réaction et la solution de BSA sont de New England Biolabs Inc. La réaction est poursuivie 3 heures au bain marie à 37°C. Le vecteur digéré est purifié grâce au coffret QIAquick Gel Extraction (Qiagen) selon les recommandations du fournisseur. Le vecteur est ensuite déphosphorylé par la CIP (New England Biolabs Inc.) pendant 1 heure à 37°C. La composition du milieu réactionnel est la suivante : tampon NEB3 10X (5 μL), vecteur (1 μg), CIP (1μL).

(b). Extraction de l'ARN total de l'hybridome 8B6, producteur de l'anticorps monoclonal 8B6.

L'ARN total est extrait à partir de 106 cellules d'hybridome 8B6 en phase exponentielle de croissance grâce au réactif RNAble (Eurobio, Courtaboeuf, France) selon les recommandations du fournisseur. La concentration en ARN total est déterminée par mesure de la densité optique à 260 nm.

(c). Obtention de la séquence nucléotidique de l'ADNc 8B6 L-VL

L'amplification génique de l'ADNc codant la région variable 8B6 L-VL a été obtenue à partir des ARN messagers par RACE-PCR de façon à obtenir la séquence nucléotidique codant le peptide signal (L) associé à sa région variable (VL). Cette amplification a été réalisée en utilisant le coffret SMARTTM RACE cDNA Amplification obtenu auprès de la société BD Biosciences (San Jose, CA, USA) selon les recommandations du fournisseur. La quantité d'ARN total utilisé pour la rétrotranscription est de 1μg. Le produit de la réaction a été dilué dans 100μL de solution tampon tricine EDTA fourni par le fournisseur. Un volume de 2,5μL du produit dilué a été utilisé pour l'amplification génique. L'amorce antisens spécifique de l'ADNc 8B6 VL utilisée est la suivante: 3'- mu C kappa 5'- gtt cat act cgt cct tgg tca acg tga ggg -3' qui s'hybride sur l'ADNc codant le domaine constant mu C-kappa de la chaîne légère des anticorps de souris de type kappa. L'amplification a été réalisée par incubation du mélange réactionnel dans un thermocycleur Perkin-Elmer (PE) DNA thermal Cycler 480 (Perkin Elmer Wellesley, MA, USA) dans les conditions suivantes : 5 cycles (94°C pendant 5 secondes, 72°C pendant 3 minutes), suivi de 5 cycles (94°C pendant 5 secondes puis 70°C pendant 10 secondes suivi de 3 minutes à 72°C) et de 25 cycles (94°C pendant 5 secondes suivi de 69°C pendant 10 secondes et 3 minutes à 72°C). Le produit de réaction de RACE-PCR est analysé par électrophorèse sur gel d'agarose 1% (Q. Biogene, Morgan Irvine, CA, USA) dans une solution tampon de migration de Tris EDTA pH 8 (40 mM Tris-base (Sigma Chemicals Co), 25 mM EDTA (Inerchim, Montluçon, France), 20 mM acide acétique (Carlo Erba Reagenti SpA, Rodano, MI, Italie). Les produits de poids moléculaire attendu sont ensuite purifiés à l'aide du coffret QIAquick Gel Extraction (Qiagen) selon les recommandations du fournisseur. La détermination de la séquence en acide nucléique des produits obtenus purifiés a été réalisée par la société GENOME express (Meylan, France) afin de vérifier la séquence de l'ADNc codant la région 8B6 L-VL. La séquence correspondant au peptide signal associé à la région variable de la chaîne légère de l'anticorps 8B6 a été ainsi déterminée et des amorces dessinées pour permettre le clonage de l'ADNc 8B6 L-VL dans le vecteur d'expression.

(d). Amplification de l'ADNc 8B6 L-VL

L'amplification de l'ADNc 8B6 L VL a été obtenue par réaction de RT-PCR à partir d'un extrait d'ARN total de cellules d'hybridome 60C3. Le mélange réactionnel est le suivant : Oligo d(T)18 0,5μg (New England Biolabs Inc. Beverly, MA, USA), ARN 1μg, dNTP 0,5 mM (Promega, Madison, WI, USA), eau stérile qsp 12μL. Ce mélange est incubé 5 minutes à 65°C (bain marie sec) puis 2 minutes à 4°C (glace fondante) pour dénaturer l'ARN. Sont ensuite ajoutés au mélange réactionnel, 4μL de solution tampon 5X First-strand Buffer (Invitrogen Life Biotechnologies), 10 mM DTT (Invitrogen Life Biotechnologies), 160 U de Rnasine (Promega) et 800 U de transcriptase inverse (Invitrogen Life Biotechnologies). L'ensemble est incubé 1 heure à 37°C puis à 70°C pendant 15 minutes afin d'arrêter la réaction. Les copies d'ADNc 8B6 L-VL sont obtenues par amplification génique grâce à une réaction de PCR utilisant les oligonucléotides de synthèse sont les suivantes : 5'- BamHI 8B6 L-VL : 5'- aag gga tcc gcc acc atg aag ttg cct gtt -3' (amorce sens) et 3'- XhoI 8B6 L-VL : 5'- ccg ttt tat ctc gag ctt ggt ccc -3' (amorce anti-sens)

Les amorces de synthèse utilisées permettent l'introduction de mutations silencieuses dans la séquence d'ADN qui n'entraînent aucune modification au niveau de la séquence en acides aminés tout en permettant l'apparition des sites de restriction BamHI et XhoI nécessaires au clonage dans le vecteur.

Le milieu réactionnel a la composition suivante : dNTP (Promega) 500 μM, Taq polymerase (Invitrogen Life Technologies) 1μL, solution tampon de PCR (Invitrogen Life Technologies) 10μL, ADNc (matrice) 1μL, MgCl2 (Invitrogen Life Technologies) 1,5 mM, amorce 3'- XhoI 8B6 L-VL et amorce : 5'- BamHI 8B6 L-VL 500μM.

L'amplification a été réalisée dans un thermocycleur MJ-research PTC 200 (Peltier Thermal Cycler) selon les conditions suivantes : 5 minutes à 94°C, puis 35 cycles suivants 30 secondes à 94°C, 45 secondes à 55°C et 1 minute à 72°C.

Après vérification des produits de PCR par analyse électrophorétique sur gel d'agarose à 1%, le cDNA 8B6 L-VL est purifié à l'aide du coffret QIAquick Gel Extraction (Qiagen) selon les recommandations du fournisseur.

(e). Digestion de l'ADNc 8B6 L-VL

L'ADNc 8B6 L-VL est digéré utilisant les enzymes de restriction BamHI et XhoI. La composition du milieu réactionnel est la suivante : tampon NEB2 10X (5 μL), BSA 100X (0,5 μL), ADNc 8B6 L-VL (1 μg), enzyme de restriction BamHI (10U), enzyme de restriction XhoI (10U). Les enzymes de restriction, les solutions tampons de réaction et la solution de BSA sont de New England Biolabs Inc. La réaction est poursuivie 3 heures au bain marie à 37°C. L'insert digéré est purifié grâce au coffret QIAquick Gel Extraction (Qiagen) selon les recommandations du fournisseur.

(f). Réaction de ligation

La ligation permet d'insérer l'ADNc 8B6 L-VL digéré dans le vecteur pcDNA3® hu-C kappa digéré et déphosphorylé grâce à la T4 ligase (New England Biolabs Inc.). La réaction est poursuivie au bain marie à 16°C pendant 16 heures dans le millieu réactionnel suivant : solution tampon de ligature 5X (4 µL) (New England Biolabs Inc.), vecteur pcDNA3® hu-C kappa digéré et déphosphorylé 200 ng, T4 ligase (400U) (New England Biolabs Inc.), ADNc 8B6 L-VL purifié 170 ng (pour un insert de 1 kb). Le rapport molaire insert/vecteur est de 3/1. Le produit de réaction de ligature est utilisé pour la transformation de bactéries compétentes E. coli XLI blue (Stratagene).

(g). Vérification de la présence de l'insert 8B6 L-VL dans le vecteur pcDNA3® hu-C kappa par réaction de PCR

Les colonies résistantes isolées sont déposées stérilement sous hotte dans un tube contenant le mélange réactionnel de PCR : dNTP (Promega) 500 µM, Taq polymerase (Invitrogen Life Technologies) 1µL, solution tampon de PCR (Invitrogen Life Technologies) 10µL, ADNc (matrice) 1µL, MgC12 (Invitrogen Life Technologies) 1,5 mM, amorce 3'- XhoI 8B6 L-VL et amorce 5'- BamHI 8B6 L-VL 500µM. L'amplification a été réalisée dans un thermocycleur MJ-research PTC 200 selon les conditions suivantes : 5 minutes à 94°C, puis 35 cycles suivants 30 secondes à 94°C, 45 secondes à 55°C et 1 minute à 72°C. La taille du produit PCR amplifié est contrôlée par analyse électrophorétique sur gel d'agarose 1%. Les clones qui ont été correctement transformés sont les seuls à donner un produit de PCR de taille attendue.

(h). Production du plasmide d'intérêt pcDNA3® KM8B6 L

On utilise un cure-dent stérile qui après prélèvement de la colonie est mis dans 5 mL de milieu liquide sélectif LB contenant 100µg/mL d'ampicilline (Sigma Chemicals Co) et 12,5µg/mL de tétracycline (Sigma Chemicals Co) et incubé 16 heures à 37°C. La dilution bactérienne est alors utilisée pour la réalisation d'une minipréparation d'ADN plasmidique en utilisant le coffret QIAprep spin miniprep (QIAGEN) selon les recommandations du fournisseur.

6. Préparation de l'ADNc codant la région variable L-VH de l'anticorps monoclonal de souris 60C3

(a). Obtention de la séquence nucléotidique de l'ADNc 60C3 L-VH

L'amplification génique de l'ADNc codant la région variable 60C3 L-VH a été obtenue à partir des ARN messagers par RACE-PCR de façon à obtenir la séquence nucléotidique codant le peptide signal (L) associé à sa région variable (VL). Cette amplification a été réalisée en utilisant le coffret SMARTTM RACE cDNA Amplification obtenu auprès de la société BD Biosciences (San Jose, CA, USA) selon les recommandations du fournisseur. La quantité d'ARN total utilisé pour la rétrotranscription est de 1µg. Le produit de la réaction a été dilué dans 100µL de solution tampon tricine EDTA fourni par le fournisseur. Un volume de 2,5µL du produit dilué a été utilisé pour l'amplification génique. L'amorce antisens spécifique de l'ADNc 60C3 VH utilisée est la suivante: 3'- 60C3 L-VH 5'-tgc AGA gac agt gac cag cag agt agt ccc -3' (amorce anti-sens) qui s'hybride sur l'ADNc codant le domaine constant mu C-kappa de la chaîne légère des anticorps de souris de type kappa.. L'amplification a été réalisée par incubation du mélange réactionnel dans un thermocycleur Perkin-Elmer (PE) DNA thermal Cycler 480 (Perkin Elmer Wellesley, MA, USA) dans les conditions suivantes : 5 cycles (94°C pendant 5 secondes, 72°C pendant 3 minutes), suivi de 5 cycles (94°C pendant 5 secondes puis 70°C pendant 10 secondes suivi de 3 minutes à 72°C) et de 25 cycles (94°C pendant 5 secondes suivi de 69°C pendant 10 secondes et 3 minutes à 72°C). Le produit de réaction de RACE-PCR est analysé par électrophorèse sur gel d'agarose 1 % (Q. Biogene, Morgan Irvine, CA, USA) dans une solution tampon de migration de Tris EDTA pH 8 (40 mM Tris-base (Sigma Chemicals Co), 25 mM EDTA (Inerchim, Montluçon, France), 20 mM acide acétique (Carlo Erba Reagenti SpA, Rodano, MI, Italie). Les produits de poids moléculaire attendu sont ensuite purifiés à l'aide du coffret QIAquick Gel Extraction (Qiagen) selon les recommandations du fournisseur. La détermination de la séquence en acide nucléique des produits obtenus purifiés a été réalisée par la société GENOME express (Meylan, France) afin de vérifier la séquence de l'ADNc codant la région 60C3 L-VH. La séquence correspondant au peptide signal associé à la région variable de la chaîne légère de l'anticorps 60C3 a été ainsi déterminée et des amorces dessinées pour permettre le clonage de l'ADNc 60C3 L-VH dans le vecteur d'expression.

(b). Amplification de l'ADNc 60C3 L-VH

L'amplification de l'ADNc 60C3 L-VH a été obtenue par réaction de RT-PCR à partir d'un extrait d'ARN total de cellules d'hybridome 60C3. Le mélange réactionnel est le suivant : Oligo d(T)18 0,5µg (New England Biolabs Inc. Beverly, MA, USA), ARN 1µg, dNTP 0,5 mM (Promega, Madison, WI, USA), eau stérile qsp 12µL. Ce mélange est incubé 5 minutes à 65°C (bain marie sec) puis 2 minutes à 4°C (glace fondante) pour dénaturer l'ARN. Sont ensuite ajoutés au mélange réactionnel, 4μL de solution tampon 5X First-strand Buffer (Invitrogen Life Biotechnologies), 10 mM DTT (Invitrogen Life Biotechnologies), 160 U de Rnasine (Promega) et 800 U de transcriptase inverse (Invitrogen Life Biotechnologies). L'ensemble est incubé 1 heure à 37°C puis à 70°C pendant 15 minutes afin d'arrêter la réaction. Les copies d'ADNc 60C3 L-VH sont obtenues par amplification génique grâce à une réaction de PCR utilisant les oligonucléotides de synthèse sont les suivantes : 5'- BamHI 60C3 L-VH : 5'- cag gat ccg aac aca ctg act cta acc atg g - 3' (amorce sens) et 3'- Nhel 60C3 L-VH : 5'- t gct agc tgc aga gac agt gac cag agt -3' (amorce anti-sens).

Les amorces de synthèse utilisées permettent l'introduction de mutations silencieuses dans la séquence d'ADN qui n'entraînent aucune modification au niveau de la séquence en acides aminés tout en permettant l'apparition des sites de restriction BamHI et XhoI nécessaires au clonage dans le vecteur.

Le milieu réactionnel a la composition suivante : dNTP (Promega) 500 μM, Taq polymerase (Invitrogen Life Technologies) 1 μL, solution tampon de PCR (Invitrogen Life Technologies) 10μL, ADNc (matrice) 1 μL, MgC12 (Invitrogen Life Technologies) 1,5 mM, amorce 3'- Nhel 60C3 L-VH et amorce 5'- BamHI 60C3 L-VH 500μM.

L'amplification a été réalisée dans un thermocycleur MJ-research PTC 200 (Peltier Thermal Cycler) selon les conditions suivantes : 5 minutes à 94°C, puis 35 cycles suivants 30 secondes à 94°C, 45 secondes à 55°C et 1 minute à 72°C.

Après vérification des produits de PCR par analyse électrophorétique sur gel d'agarose à 1%, le cDNA 60C3 L-VH est purifié à l'aide du coffret QIAquick Gel Extraction (Qiagen) selon les recommandations du fournisseur.

7. Construction du plasmide recombinant possédant l'ADNc 60C3 L-VH

(a). Construction du vecteur de clonage pBluescript II SK (+) 60C3 L-VH

Pour insérer la séquence codant la région 60C3 L-VH dans le vecteur pBluescript II SK (+), ce dernier est digéré par l'enzyme de restriction EcoRV 'New England Biolabs Inc.) qui provoque une coupure à bouts francs. Le traitement du plasmide ainsi linéarisé par la Taq polymerase en présence de dTTP permet de rajouter une thymidine à l'extrémité 3' du vecteur et empêche l'autoligature. La Taq DNA polymérase utilisée pour l'obtention de l'ADNc 60C3 L-VH possède une activité 5'-3' exonucléase ajoutant une adénine terminale à chaque extrémité 3' des produits de PCR qui peut être directement cloné dans un tel vecteur. Le rendement de le réaction de ligature est largement amélioré du fait de la complémentarité A/T à celui de la réaction de ligature à bout franc. Le produit de réaction de ligature est utilisé pour la transformation de bactéries compétentes E. coli XL 1 blue (Stratagene).

(b). Vérification de la présence de l'insert 60C3 L-VH dans le vecteur pBluescript II SK (+) par réaction de PCR

Les colonies résistantes isolées sont déposées stérilement sous hotte dans un tube contenant le mélange réactionnel de PCR : dNTP 500 μM (Promega), Taq polymerase (Invitrogen Life Technologies) 1μL, solution tampon de PCR (Invitrogen Life Technologies) 10μL, MgC12 (Invitrogen Life Technologies) 1,5 mM, amorce 3'- Nhel 60C3 L-VH et amorce 5'- BamHI 60C3 L-VH 500μM.. L'amplification a été réalisée dans un thermocycleur MJ-research PTC 200 selon les conditions suivantes : 5 minutes à 94°C, puis 35 cycles suivants 30 secondes à 94°C, 45 secondes à 55°C et 1 minute à 72°C. La taille du produit PCR amplifié est contrôlée par analyse électrophorétique sur gel d'agarose 1%. Les clones qui ont été correctement transformés sont les seuls à donner un produit de PCR de taille attendue.

(c). Production du plasmide d'intérêt pBluescript II SK (+)60C3 L-VH

Pour cela, on utilise un cure-dent stérile qui après prélèvement de la colonie est mis dans 5 mL de milieu liquide sélectif LB contenant 100μg/mL d'ampicilline (Sigma Chemicals Co) et 12,5μg/mL de tétracycline (Sigma Chemicals Co) et incubé 16 heures à 37°C. La dilution bactérienne est alors utilisée pour la réalisation d'une minipréparation d'ADN plasmidique en utilisant le coffret QIAprep spin miniprep (QIAGEN) selon les recommandations du fournisseur.

(d). Vérification de l'orientation de l'insert 60C3 L-VH dans le vecteur pBluescript II SK (+) 60C3 L-VH par digestion enzymatique

La présence de l'insert 60C3 L-VH en orientation sens est confirmée par une digestion enzymatique du vecteur pBluescript II SK (+) 60C3 L-VH par l'enzyme BamHI (New England Biolabs Inc.) selon les recommandations du fournisseur. En effet, la technique de clonage T n'impose pas d'orientation pour l'insert qui peut donc être inséré en orientation sens ou antisens. L'insert 60C3 L-VH n'est encadré par deux sites de restriction BamHI que s'il est en orientation sens. Ainsi après digestion par l'enzyme BamHI, l'analyse électrophorétique permet de distinguer et de sélectionner un clone possédant l'ADNc 60C3 L-VH en orientation sens.

8. Préparation de l'ADNc codant la région constante d'un anticorps humain hu-C gamma 1

a. Extraction de l'ARN total de la lignée de myélome humain LP1 producteur d'une chaîne lorude d'isotype gamma 1.

L'ARN total est extrait à partir de 106 cellules d'hybridome LP1 en phase exponentielle de croissance grâce au réactif RNAble (Eurobio, Courtaboeuf, France) selon les recommandations du fournisseur. La concentration en ARN total est déterminée par mesure de la densité optique à 260 nm.

b. Amplification génique du segment ADNc hu-C gamma 1

L'amplification de l'ADNc hu-C gamma 1 a été réalisée à partir d'un extrait d'ARN total de l'hybridome LP1. Le mélange réactionnel est le suivant : Oligo d(T)18 0,5μg (New England Biolabs Inc. Beverly, MA, USA), ARN 1μg, dNTP 0,5 mM (Promega, Madison, WI, USA), eau stérile qsp 12μL. Ce mélange est incubé 5 minutes à 65°C (bain marie sec) puis 2 minutes à 4°C (glace fondante) pour dénaturer l'ARN. Sont ensuite ajoutés au mélange réactionnel, 4μL de solution tampon 5X First-strand Buffer (Invitrogen Life Biotechnologies), 10 mM DTT (Invitrogen Life Biotechnologies), 160 U de Rnasine (Promega) et 800 U de transcriptase inverse (Invitrogen Life Biotechnologies). L'ensemble est incubé 1 heure à 37°C puis à 70°C pendant 15 minutes afin d'arrêter la réaction. Les copies d'ADNc ADNc hu-C gamma 1 sont obtenues par amplification génique grâce à une réaction de PCR utilisant les oligonucléotides de synthèse sont les suivantes : 5'- NheI hu-C gamma 1: 5'- ca gct agc acc aag ggc cca tcg gtc ttc c -3' (amorce sens) et 3'- XbaI hu-C gamma 1: 5'- agc ctc tcc ctg tct ccg ggt aaa taa tct aga cg -3' (amorce anti-sens).

Les amorces de synthèse utilisées permettent l'introduction de mutations silencieuses dans la séquence d'ADN qui n'entraînent aucune modification au niveau de la séquence en acides aminés tout en permettant l'apparition des sites de restriction NheI et XbaI nécessaires au clonage dans le vecteur.

Le milieu réactionnel a la composition suivante : dNTP (Promega) 500 μM, Taq polymerase (Invitrogen Life Technologies) 1μL, solution tampon de PCR (Invitrogen Life Technologies) 10μL, ADNc (matrice) 1μL, MgCl2 (Invitrogen Life Technologies) 1,5 mM, amorce 3'- XbaI hu-C gamma 1 et amorce 5'- NheI hu-C gamma 1500μM.

L'amplification a été réalisée dans un thermocycleur MJ-research PTC 200 (Peltier Thermal Cycler) selon les conditions suivantes : 5 minutes à 94°C, puis 35 cycles suivants 30 secondes à 94°C, 45 secondes à 55°C et 1 minute à 72°C.

Après vérification des produits de PCR par analyse électrophorétique sur gel d'agarose à 1%, le cDNA hu-C gamma 1 est purifié à l'aide du coffret QIAquick Gel Extraction (Qiagen) selon les recommandations du fournisseur.

9. Construction d'un plasmide recombinant possédant l'ADNc 60C3 L-VH couplé à l'ADNc hu-C gamma 1 : pBluescript II SK (+) KM60C3 H

(a). Digestion du vecteur pBluescript II SK (+) 60C3 L-VH et de l'ADNc hu-C gamma 1 et déphosphorylation du vecteur digéré

La digestion se fait dans les conditions suivantes : tampon NEB2 10X (5 μL), BSA 100X (0,5 μL), vecteur pBluescript II SK (+) 60C3 L-VH ou l'ADNc hu-C gamma 1 (1 μg), enzyme de restriction NheI (10U), enzyme de restriction XbaI (10U). Les enzymes de restriction, les solutions tampons de réaction et la solution de BSA sont de New England Biolabs Inc. La réaction est poursuivie 3 heures au bain marie à 37°C. Le vecteur et l'insert digérés sont purifiés grâce au coffret QIAquick Gel Extraction (Qiagen) selon les recommandations du fournisseur. Le vecteur est ensuite déphosphorylé par la CIP (New Englan Biolabs Inc.) pendant 1 heure à 37°c. La composition du milieu réactionnel est la suivante : tampon NEB3 10X (5 μL), vecteur (1 μg), CIP (1μL).

(b). Réaction de ligation

La ligation permet d'insérer l'ADNc hu-C gamma 1 digéré dans le vecteur pBluescript II SK (+) 60C3 L-VH digéré et déphosphorylé grâce à la T4 ligase (New England Biolabs Inc.). La réaction de ligature est poursuivie au bain marie à 16°C pendant 16 heures dans le millieu réactionnel suivant : solution tampon de ligature 5X (4 μL) (New England Biolabs Inc.), vecteur digéré déphosphorylé pBluescript II SK (+) 60C3 L-VH 200 ng, T4 ligase (400U) (New England Biolabs Inc.), ADNc hu-C gamma 1 purifié 170 ng (pour un insert de 1 kb). Le rapport molaire insert/vecteur est de 3/1. Le produit de réaction de ligation est utilisé pour la transformation de bactéries compétentes E. coli XL1 blue (Stratagene).

(c). Vérification de la présence et de l'orientation de l'insert hu-C gamma 1 dans le vecteur pBluescript II SK (+) 60C3 L-VH par réaction de PCR

En raison du choix des sites de restriction NheI et XbaI, l'insert hu-C gamma 1 peut être dans l'orientation sens ou antisens. Ceci est dû à la séquence des sites de restrction qui n'impose pas d'orientation pour l'insert. L'orientation sens de l'insert est confirmée par une réaction de PCR. Des colonies isolées sont déposées stérilement sous hotte respectivement dans un tube contenant le mélange réactionnel de PCR : dNTP 500 $\mu$M (Promega), Taq polymerase (Invitrogen Life Technologies) 1$\mu$L, solution tampon de PCR (Invitrogen Life Technologies) 10$\mu$L, MgC12 (Invitrogen Life Technologies) 1,5 mM, amorce sens et amorce antisens 500$\mu$M. L'amplification a été réalisée dans un thermocycleur MJ-research PTC 200 selon les conditions suivantes : 5 minutes à 94°C, puis 35 cycles suivants 30 secondes à 94°C, 45 secondes à 55°C et 1 minute à 72°C. Les amorces utilisées sont :5'- BamHI 60C3 L-VH : 5'- cag gat ccg aac aca ctg act cta acc atg g -3' (amorce sens) et 3'- XbaI hu-C gamma 1: 5'- agc ctc tcc ctg tct ccg ggt aaa taa tct aga cg -3' (amorce anti-sens).

Si l'insert n'est pas dans la bonne orientation, les amorces se retrouvent sur le même brin de la séquence à amplifier et il n'y a donc pas d'amplification. La taille du produit PCR amplifié est contrôlée par analyse électrophorétique sur gel d'agarose 1%. Les clones qui ont été correctement transformés sont les seuls à donner un produit de PCR de taille attendue.

(d). Production du plasmide d'intérêt pBluescript II SK (+) KM60C3 H

On utilise un cure-dent stérile qui après prélèvement de la colonie est mis dans 5 mL de milieu liquide sélectif LB contenant 100$\mu$g/mL d'ampicilline (Sigma Chemicals Co) et 12,5$\mu$g/mL de tétracycline (Sigma Chemicals Co) et incubé 16 heures à 37°C. La dilution bactérienne est alors utilisée pour la réalisation d'une minipréparation d'ADN plasmidique en utilisant le coffret QIAprep spin miniprep (QIAGEN) selon les recommandations du fournisseur.

10. Construction du vecteur d'expression de la chaîne lourde de l'anticorps modifié artificiellement KM 8B6 : pcDNA3/Hygro 8B6-H

(a). Obtention du vecteur pBluescript II SK (+) hu-C gamma 1

Le vecteur pBluescript II SK (+) hu-C gamma la été préparé à partir du vecteur pBluescript II SK (+) KM60C3 H par digestion utilisant les enzymes de restriction BamHI et XbaI. La composition du milieu réactionnel est la suivante : tampon NEB2 10X (5 $\mu$L), BSA 100X (0,5 $\mu$L), vecteur (1 $\mu$g), enzyme de restriction BamHI (10U), enzyme de restriction XbaI (10U). Les enzymes de restriction, les solutions tampons de réaction et la solution de BSA sont de New England Biolabs Inc. La réaction est poursuivie 3 heures au bain marie à 37°C. Le vecteur est alors purifié grâce au coffret QIAquick Gel Extraction (Qiagen) selon les recommandations du fournisseur. Le vecteur est ensuite déphosphorylé par la CIP (New England Biolabs Inc.) pendant 1 heure à 37°C. La composition du milieu réactionnel est la suivante : tampon NEB3 10X (5 $\mu$L), vecteur ( 1$\mu$g), CIP (1$\mu$L).

(b). Extraction de l'ARN total de l'hybridome 8B6 producteur de l'anticorps monoclonal 8B6.

L'ARN total est extrait à partir de 106 cellules d'hybridome 8B6 en phase exponentielle de croissance grâce au réactif RNAble (Eurobio, Courtaboeuf, France) selon les recommandations du fournisseur. La concentration en ARN total est déterminée par mesure de la densité optique à 260 nm.

(c). Obtention de la séquence nucléotidique du cDNA 8B6 L-VH

L'amplification génique de l'ADNc codant la région variable 8B6 L-VH a été obtenue à partir des ARN messagers par RACE-PCR de façon à obtenir la séquence nucléotidique codant le peptide signal (L) associé à sa région variable (VH). Cette amplification a été réalisée en utilisant le coffret SMART RACE cDNA Amplification obtenu auprès de la société BD Biosciences (San Jose, CA, USA) selon les recommandations du fournisseur. La quantité d'ARN total utilisé pour la rétrotranscription était de 1$\mu$g. Le produit de la réaction a été dilué dans 100$\mu$L de solution tampon tricine EDTA fourni par le fournisseur. Un volume de 2,5$\mu$L du produit dilué a été utilisé pour l'amplification génique. L'amorce antisens spécifique de l'ADNc 8B6 L-VH utilisée est la suivante : 3'- mu C gamma 3 5'- tga tca act cag tct tgc tgg ctg ggt ggg -3' qui s'hybride sur l'ADNc codant le domaine constant mu C-gamma 3 de la chaîne lourde des anticorps de souris de type gamma 3. L'amplification a été réalisée par incubation du mélange réactionnel dans un thermocycleur Perkin-Elmer (PE) DNA thermal Cycler 480 (Perkin Elmer Wellesley, MA, USA) dans les conditions suivantes : 5 cycles (94°C pendant 5 secondes, 72°C pendant 3 minutes), suivi de 5 cycles (94°C pendant 5 secondes puis 70°C pendant 10 secondes suivi de 3 minutes à 72°C) et de 25 cycles (94°C pendant 5 secondes suivi de 69°C pendant 10 secondes et 3 minutes à 72°C). Le produit de réaction de RACE-PCR est analysé par électrophorèse sur gel d'agarose 1 % (Q. Biogene, Morgan Irvine, CA, USA) dans une solution tampon de migration de Tris EDTA pH 8 (40 mM Tris-base (Sigma Chemicals Co), 25 mM EDTA (Inerchim, Montluçon, France), 20 mM acide acétique (Carlo Erba Reagenti SpA, Rodano, MI, Italie). Les produits de poids moléculaire attendu sont ensuite purifiés à l'aide du coffret QIAquick Gel Extraction (Qiagen) selon les recommandations du fournisseur. La détermination de la séquence en acide nucléique des produits obtenus purifiés a été réalisée par la société GENOME express (Meylan, France) afin de vérifier la séquence de l'ADNc codant la région 8B6 L-VH. La séquence correspondant au peptide signal associé à la région variable de la chaîne lourde de l'anticorps 8B6 a été ainsi déterminée et des amorces dessinées pour permettre le clonage de l'ADNc 8B6 L-VH dans le vecteur d'expression pBluescript II SK (+) hu-C gamma 1.

(d). amplification génique des segments 8B6 L-VH

L'amplification de l'ADNc 8B6 L-VH a été obtenue par réaction de RT-PCR à partir d'un extrait d'ARN total de cellules d'hybridome 8B6. Le mélange réactionnel est le suivant: Oligo d(T)18 1μL (New England Biolabs Inc. Beverly, MA, USA), ARN 1μg, dNTP 0,5 mM (Promega, Madison, WI, USA), eau stérile qsp 12μL. Ce mélange est incubé 5 minutes à 65°C (bain marie sec) puis 2 minutes à 4°C (glace fondante) pour dénaturer l'ARN. Sont ensuite ajoutés au mélange réactionnel, 4μL de solution tampon 5X First-strand Buffer (Invitrogen Life Biotechnologies), 10 mM DTT (Invitrogen Life Biotechnologies), 160 U de Rnasine (Promega) et 800 U de transcriptase inverse (Invitrogen Life Biotechnologies). L'ensemble est incubé 1 heure à 37°C puis à 70°C pendant 15 minutes afin d'arrêter la réaction. Les copies d'ADNc 8B6 L-VH sont obtenues par amplification génique grâce à une réaction de PCR utilisant les oligonucléotides de synthèse suivants : 5'- BamHI 8B6 L-VH : 5'-ccg tcg gat ccg gcc acc atg aag ttg tgg -3' (amorce sens) et 3'- NheI 8B6 L-VH : 5'-cgg ggt gct agc tga gga gac tgt -3' (amorce antisens).

Les amorces utilisées permettent l'introduction de mutations silencieuses dans la séquence d'ADN qui n'entraînent aucune modification au niveau de la séquence en acides aminés tout en permettant l'apparition des sites de restriction BamHI et NheI nécessaires au clonage dans le vecteur.

Le milieu réactionnel a la composition suivante : dNTP 500 μM (Promega), Taq polymerase (Invitrogen Life Technologies) 1μL, solution tampon de PCR (Invitrogen Life Technologies) 10μL, ADNc (matrice) 1μL, MgCl2 (Invitrogen Life Technologies) 1,5 mM, amorce 3'- NheI 8B6 L-VH et amorce 5'- BamHI 8B6 L-VH 500μM.

L'amplification a été réalisée dans un thermocycleur MJ-research PTC 200 (Peltier Thermal Cycler) selon les conditions suivantes : 5 minutes à 94°C, puis 35 cycles suivants 30 secondes à 94°C, 45 secondes à 55°C et 1 minute à 72°C.

Après vérification des produits de PCR par analyse électrophorétique sur gel d'agarose à 1%, le cDNA 8B6 L-VH est purifié à l'aide du coffret QIAquick Gel Extraction (Qiagen) selon les recommandations du fournisseur.

(e). Digestion de l'ADNc 8B6 L-VH

L'ADNc 8B6 L-VH est digéré utilisant les enzymes de restriction BamHI et NheI. La composition du milieu réactionnel est la suivante : tampon NEB2 10X (5 μL), BSA 100X (0,5 μL), ADNc 8B6 L-VH (1 μg), enzyme de restriction BamHI (10U), enzyme de restriction NheI (10U). Les enzymes de restriction, les solutions tampons de réaction et la solution de BSA sont de New England Biolabs Inc. La réaction est poursuivie 3 heures au bain marie à 37°C. L'insert digéré est purifié grâce au coffret QIAquick Gel Extraction (Qiagen) selon les recommandations du fournisseur.

(f). Réaction de ligation

La ligation permet d'insérer l'ADNc 8B6 L-VH digéré dans le vecteur pBluescript II SK (+) hu-C gamma 1 digéré et déphosphorylé. La réaction est poursuivie au bain marie à 16°C pendant 16 heures dans le milieu réactionnel suivant : solution tampon de ligature 5X (4 μL) (New England Biolabs Inc.), vecteur digéré déphosphorylé pBluescript II SK (+) hu-C gamma 1 200 ng, T4 ligase (400U) (New England Biolabs Inc.), ADNc 8B6 L-VH purifié 170 ng (pour un insert de 1 kb). Le rapport molaire insert/vecteur est de 3/1. Le produit de réaction de ligature est utilisé pour la transformation de bactéries comp étentes E. coli XL1 blue (Stratagene).

(g). Obtention de l'ADNc codant la chaîne lourde de l'anticorps modifié artificiellement

Le vecteur pBluescript II SK (+) KM8B6 H est digéré par les enzymes de restriction BamHI et XbaI pour obtenir l'ADNc codant la chaîne lourde de l'anticorps modifié artificiellement. La composition du milieu réactionnel est la suivante : tampon NEB2 10X (5 μL), BSA 100X (0,5 μL), vecteur (1 μg), enzyme de restriction BamHI (10U), enzyme de restriction XbaI (10U). Les enzymes de restriction, les solutions tampons de réaction et la solution de BSA sont de New England Biolabs Inc. La réaction est poursuivie 3 heures au bain marie à 37°C. L'insert libéré est purifié grâce au coffret QIAquick Gel Extraction (Qiagen) selon les recommandations du fournisseur.

(h). Digestion du vecteur pcDNA3/Hygro et déphosphorylation du vecteur digéré

La digestion se fait dans les conditions suivantes : tampon NEB2 10X (5 μL), BSA 100X (0,5 μL), vecteur (1 μg), enzyme de restriction BamHI (10U), enzyme de restriction XbaI (10U). Les enzymes de restriction, les solutions tampons de réaction et la solution de BSA sont de New England Biolabs Inc. La réaction est poursuivie 3 heures au bain marie à 37°C. Le vecteur et l'insert digérés sont purifiés grâce au coffret QIAquick Gel Extraction (Qiagen) selon les recommandations du fournisseur. Le vecteur est ensuite déphosphorylé par la CIP (New Englan Biolabs Inc.) pendant 1 heure à 37°c. La composition du milieu réactionnel est la suivante: tampon NEB3 10X (5 μL), vecteur (1 μg), CIP (1 μL).

(i). Réaction de ligation

La réaction de ligation permet d'insérer l'ADNc codant la chaîne lourde de l'anticorps modifié artificiellement dans le vecteur pcDNA3/Hygro digéré et déphosphorylé. La réaction de ligature est poursuivie au bain marie à 16°C pendant 16 heures dans le millieu réactionnel suivant : solution tampon de ligature 5X (4 μL) (New England Biolabs Inc.), vecteur digéré déphosphorylé pcDNA3/Hygro 200 ng, T4 ligase (400U) (New England Biolabs Inc.), l'ADNc codant la chaîne lourde de l'anticorps modifié artificiellement purifié 170 ng (pour un insert de 1 kb). Le rapport molaire insert/ vecteur est de 3/1. Le produit de réaction de ligation est utilisé pour la transformation de bactéries compétentes E. coli XL1 blue (Stratagene).

(j). Vérification de la présence de l'ADNc codant la chaîne lourde de l'anticorps modifié artificiellement dans le vecteur pcDNA3/Hygro par réaction de PCR

Les colonies résistantes isolées sont déposées stérilement sous hotte dans un tube contenant le mélange réactionnel de PCR : dNTP 500 μM (Promega), Taq polymerase (Invitrogen Life Technologies) 1 μL, solution tampon de PCR (Invitrogen Life Technologies) 10μL, MgC12 (Invitrogen Life Technologies) 1,5 mM, amorce 3' et amorce 5' 500μM. L'amplification a été réalisée dans un thermocycleur MJ-research PTC 200 selon les conditions suivantes : 5 minutes à 94°C, puis 35 cycles suivants 30 secondes à 94°C, 45 secondes à 55°C et 1 minute à 72°C. Les amorces utilisées sont : 5'- BamHI 60C3 L-VH : 5'- ccg tcg gat ccg gcc acc atg aag ttg tgg -3' (amorce sens) et 3'- Nhel 8B6 L-VH: 5'- cgg ggt gct agc tga gga gac tgt -3' (amorce antisens).
La taille du produit PCR amplifié est contrôlée par analyse électrophorétique sur gel d'agarose 1%. Les clones qui ont été correctement transformés sont les seuls à donner un produit de PCR de taille attendue.

(k). Production du plasmide d'intérêt pcDNA3 KM8B6 L

On utilise un cure-dent stérile qui après prélèvement de la colonie est mis dans 5 mL de milieu liquide sélectif LB contenant 100μg/mL d'ampicilline (Sigma Chemicals Co) et 12,5μg/mL de tétracycline (Sigma Chemicals Co) et incubé 16 heures à 37°C. La dilution bactérienne est alors utilisée pour la réalisation d'une minipréparation d'ADN plasmidique en utilisant le coffret QIAprep spin miniprep (QIAGEN) selon les recommandations du fournisseur.

11. Transfection des cellules CHO pour l'expression de l'anticorps modifié artificiellement KM8B6

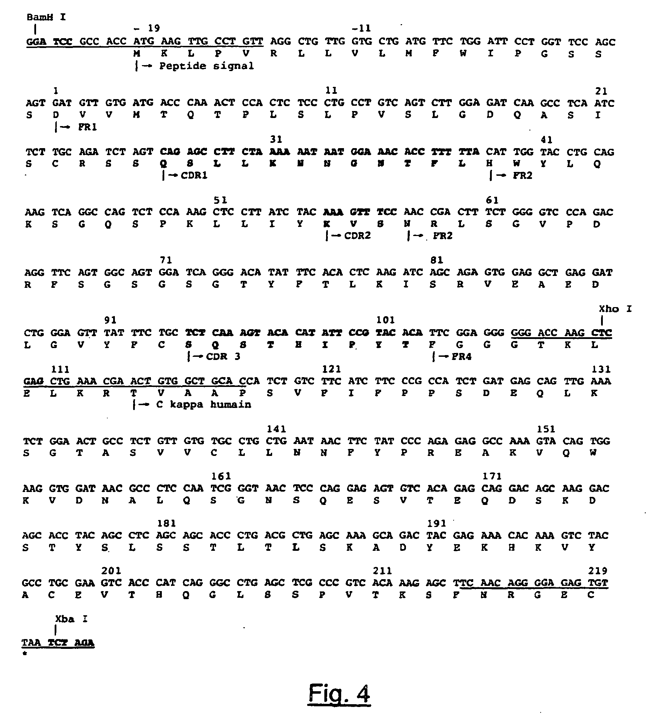

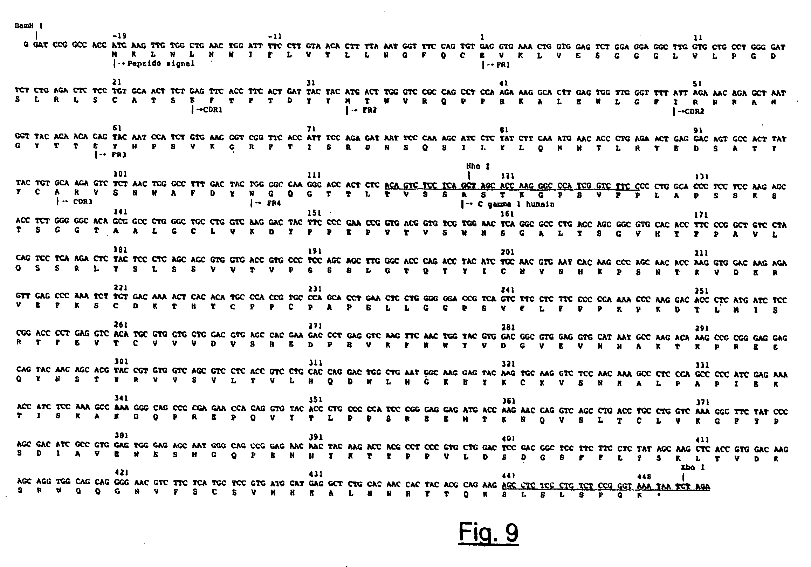

Les cellules CHO ont été utilisées comme cellules hôtes pour exprimer et sécréter l'anticorps modifié artificiellement KM8B6. Ces cellules ont été co-transféctées avec les plasmides pDNA3® KM8B6-L et pDNA3.1/Hygro© KM8B6-H, codant respectivement la chaîne légère (Figure 4) et la chaîne lourde de l'anticorps modifié artificiellement KM8B6 (Figure 9),à l'aide du coffret PolyFect® (Qiagen GMBH, Hildn, Allemagne) selon les recommandations du fournisseur. Les cellules transformées ont été sélectionnées pour leur résistance à la généticine® et l'hygromycine B® (Invitrogen Life Technologies, Carlsbad, CA, USA). Des clones résistants ont été obtenus par une technique classique de clonage par dilution limite. Ils ont été ensuite sélectionnés pour leur expression et leur sécrétion de l'anticorps KM8B6 par test immuno-enzymatique ELISA. Un clone transfectant stable sécrétant l'anticorps KM8B6 à 3,8μg/mL a été retenu.

12. Purification des anticorps 8B6 et KM8B6

Les anticorps monoclonaux de souris spécifiques des gangliosides ont été produits à partir de surnageant de culture de l'hybridome correspondant (Cerato et al, 1997). Ils ont été purifiés par chromatographie sur protéine A (GE Healthcare Amersham Bioscience AB, Uppsala, Suède) selon un protocole mis au point par l'inventeur qui limite les phénomènes d'aggrégations homophiles irréversibles des IgG3 de souris (Chapman et al, 1990). La colonne est d'abord équilibrée par un tampon 0,1 M Tris-HCl pH 7,6. Un échantillon de 2 L de surnageant de culture contenant 10% de tampon d'équilibration est déposé sur la colonne avec un débit de 1 mL/min. La colonne est ensuite lavée avec 10 volumes de tampon d'équilibration. Le matériel fixé à la protéine A est élué avec un tampon acide 0,1 M citrate 0,3 M NaCl pH 3 puis collecté par fractions immédiatement neutralisées par le dépôt préalable dans des tubes collecteurs d'un tampon 1 M Tris-HCl pH 7,6. Le déroulement de la purification est suivi par la mesure de la variation de la densité optique des fractions collectées. Les fractions intéressantes sont ensuite dialysées dans une solution de PBS pH 7,4 NaCl 0,3M et stérilisées par filtration sur filtre 0,22μm. La quantité de protéine est déterminée par mesure de la densité optique à 280 nm puis la concentration de l'AcM est ajustée à une concentration inférieure ou égale à 0,9 mg d'AcN par mL de façon à prévenir le phénomène d »aggrégation homophile des IgG3 de souris. Les AcM purifiés sont conservés ainsi à 4°C jusqu'à utilisation.

L'anticorps modifié artificiellement KM8B6 a été purifié par chromatographie d'affinité sur protéine A à partir du surnageant de culture de cellule CHO provenant d'un clone co-transfécté de manière stable avec les plasmides pcDNA3 KM8B6-L et pcDNA3/Hygro KM8B6-H.

Les anticorps purifiés sont analysés par analyse SDS-PAGE en conditions dénaturantes réductrices. Les échantillons sont repris dans une solution tampon Tris-HCl 0,5 M pH 6,8, contenant 10% de glycérol 'VWR, Fontenay sous Bois, France) et 5% de B-mercaptoethanol (Promega). Ils sont portés à ébullition 5 minutes, puis analysés en électrophorèse SDS-PAGE selon la technique de Laemmli. Les protéines sont déposées à raison de 3μg dans des puits d'un gel de polyacrylamide de 1,5 mm d'épaisseur avec pour gel supérieur et inférieur des concentrations respectives de polyacrylamide de 4,5% et 12% (VWR). Après électrophorèse à 200V pendant 45 minutes à température ambiante, le gel est fixé et coloré au bleu de Coomassie R250 (Sigma Chemicals Co). Les masses moléculaires sont calculées à partir de la migration de marqueur de poids moléculaire Precision Plus Protein Standards (BIO-RAD).

L'analyse SDS-PAGE sur gel à 12% en conditions dénaturantes réductrices montre que les masses moléculaires des chaînes chimériques L et H sont respectivement de 25 kDa et 50kDa. L'analyse SDS-PAGE sur gel à 6% en conditions dénaturantes non réductrices montre que les chaînes L et H sont correctement assemblées pour former une molécule d'anticorps et que la masse moléculaire de l'anticorps modifié artificiellement est de l'ordre de 150 kDa.

13. Etude de la spécificité de l'anticorps KM8B6

(a). par immunofluorescence indirecte sur cellules vivantes

La spécificité des anticorps a été étudiée sur les cellules IMR32 qui expriment le GD2-O-acétylé et sur les cellules Neuro 2A qui n'expriment pas le GD2-O-acétylé. Les cellules au nombre de $10^6$ sont mises à incuber avec l'anticorps 8B6 ou KM8B6 pendant 30 minutes à 4°C. Les cellules sont ensuite lavées deux fois dans du tampon PBS-BSA 1% pH 7,4 et mises à incuber de nouveau 30 minutes en présence d'anticorps de chèvre F(ab)2 anti-immunoglobulines de souris ou anti-immunoglobulines humaines marqués à l'isothiocyanate de fluorescéine (Jackson Immuno-Research Europe LTD, Cambrideshire, UK) dilués au 1/100 dans du PBS-BSA 1 %. Après trois nouveaux lavages dans du PBS, 10 000 cellules sont analysées à l'aide d'un cytomètre de flux FACScan (Beton-Dockinson, Mountain View, CA) dans une fenêtre SSC/FL1-H après exclusion des cellules mortes et des débris cellulaires. Sont déclarées positives, les cellules présentant une fluorescence supérieure à 1 Log après ajustement de la base à l'aide de témoins marqués non spécifiquement par le second anticorps seul.

Les résultats obtenus montrent que l'anticorps KM8B6, comme l'anticorps 8B6, reconnaît uniquement les cellules IMR32.

(b). par test immuno-enzymatique ELISA sur des cellules dessiquées en plaque de microtitration à 96 puits

La spécificité des anticorps a été étudiée sur les cellules IMR32 qui expriment le GD2-O-acétylé et sur les cellules Neuro 2A qui n'expriment pas le GD2-O-acétylé. Les cellules tumorales cultivées in vitro sont décollées de leur support de culture par traitement à la trypsine. Après trois lavages avec du PBS, les cellules sont distribuées dans des plaques de microtitration à fond plat Maxisorp (Nunc A/S, Roskilde, Danemark) à raison de 105 cellules par puits dans un volume de 50μL de PBS. Les plaques sont ensuite placées dans une étuve à 37°C pendant une nuit pour permettre l'évaporation du PBS. Les plaques peuvent être utilisées directement ou conservées pendant plusieurs mois à température ambiante avant utilisation. Au moment de l'analyse, les plaques sont d'abord incubées sous agitation une heure à témpéarture ambiante avec 200μL d'une solution tampon de PBS pH 7,4 contenant 1% d'e BSA afin de saturer les sites non spécifiques. La plaque est ensuite incubée durant 2 heures sous agitation à température ambiante avec 100μL d'une solution d'anticorps dilué dans du tampon PBS à 0,1% de BSA. Après trois lavages , chacun effectué avec 200μL de tampon PBS, 100μL d'une solution de F(ab')2 d'anticorps biotinylés spécifiques soit d'immunoglobulines totales de souris, soit d'immunoglobulines totales humaine (Jackson ImmunoResearch Europe LTD, Cambrideshire, UK) dilués au 1/2500 dans du PBS-BSA 0,1% est déposée dans chaque puit. Après 1 heure d'incubation sous agitation à température ambiante et trois lavages en PBS, une solution de streptavidine peroxydase biotinylée (Jackson ImmunoResearch Europe LTD, Cambrideshire, UK) dans du PBS-BSA 0,1% est mise à réagir pendant 1 heure avant d'être éliminée par lavage. La révélation du complexe fixé est mise en évidence par addition de 100µL de substrat ABTS (Roche Diagnostics GmbH Mannheim, Germany). La densité optique est déterminée par une lecture de la plaque au spectrphotomètre (Multisan EX, Thermo Electron Corporation, Waltham, MA) à 405 nm à différents intervals de temps.

Les résultats obtenus montrent que l'anticorps KM8B6, comme l'anticorps 8B6, se fixe de manière dose-dépendante exclusivement sur les cellules IMR32.

(c). sur couche mince de silice

L'analyse a été réalisée sur un extrait gangliosidique total de cellules IMR32. Les gangliosides tissulaires sont extraits selon la technique décrite par Ariga et al. (1991). L'échantillon à extraire est broyé dans dix volumes d'un mélange chloroforme/méthanol (C :M) (1 : 1, v/v) et laissé sous agitation mécanique à température ambiante pendant une nuit. Après filtration, le résidu est repris dans 2,5 volumes (C :M) (1 : 1, v/v) et à nouveau agité durant six heures. Le mélange est alors filtré et les deux filtrats sont évaporés sous pression réduite dans un évaporateur rotatif. Le résidu sec est alors déposé sur une colonne contenant 2 mL de DEAE-Sephadex A-25 sous forme acétate (Sigma Chemicals Co). Les lipides neutres sont éliminés par 15 mL de solvant A. Les gangliosides sont ensuite élués avec 15 mL de méthanol contenant 0,4M d'acétate de sodium 'Sigma Chemicals Co). A la fraction obtenue sont ajoutés 30 mL de PBS pH 7,4 pour être déssalée sur une colonne Sep-Pak (Waters Co., Milford, MA, USA) de gel hydrophobe C18 selon la méthode de Mc Luer (1990). Le gel C18 est d'abord conditionné par deux volumes de colonne de méthanol puis du mélange méthanol/PBS 1 :2 (v/v). L'extrait à dessaler est ensuite déposé sur la colonne à une vitesse de 1 mL/min. Les chaînes hydrocarbonnées des gangliosides interagissent par des liaisons hydrophobes avec le gel alors que les sels et les autres molécules non hydrophobes sont éliminés par deux volumes de colonne d'eau distillée. Les glycosphingolipides sont ensuite élués par un volume de méthanol puis un volume d'un mélange chloroforme/méthanol 2 :1 (v/v). Les gangliosides élués de cette colonne sont concentrés dans un nouveau volume adéquat d'un mélange C/M 2 :1 (v/v) et conservés à -20°C.

Les gangliosides sont ensuite séparés par chromatographie sur couche mince. Cette méthode permet d'apprécier le profil des gangliosides totaux. Les plaques HPTLC sont constituées d'un gel de silice 60 'Merck) recouvrant une feuille d'aluminium. Les gangliosides déposés migrent 20 minutes à température ambiante dans une cuve saturée en solvant de migration. Ce solvant (phase mobile) est constitué d'un mélange C/M/CaCl2 dans l'eau 0,22% (50:45:10, v/v/v). Plus un ganglioside est sialylé, plus il est polaire et moins il sera mobile. La détection des gangliosides sur plaque est réalisée chimiquement par le réactif résorcinol/HCl (Svennerholm, 1963). Ce réactif ne réagit qu'avec l'acide sialique qui est caractéristique des gangliosides. Après révélation au résorcinol, plusieurs bandes colorées sont observées correspondant à la migration des gangliosides de l'extrait total. L'identification des gangliosides séparés est réalisée soit à l'aide d'anticorps monoclonaux spécifiques, soit par comparaison avec des gangliosides standards utilisés comme marqueurs ayant migrés en même temps que l'extrait gangliosidique à analyser.

Après migration des gangliosides sur une couche mince de silice, la plaque est immergée dans une solution de poly-(isobutyl)-méthacrylate 0,01% en hexane pendant 1 minute puis séchée à l'air. Ceci permet de plastifier la plaque et d'éviter le décollement du gel de son support lors des étapes ultérieures. Le protocole du test ELISA sur cellules dessiquées est alors suivi à l'exception de la révélation de la fixation de l'anticorps au ganglioside qui est effectué avec une solution de 4-chloro-1 naphtol (Sigma Aldrich Chemie GmbH, Stenheim, germany) préparée extemporanément à raison de 1 mg de produit dissous dans 1 mL de méthanol, repris dans 20 mL de PBS et additionné de 30µL d'eau oxygénée 30 volumes.

Les résultats montrent que l'anticorps KM8B6, comme l'anticorps 8B6, reconnaît le GD2-O-acétylé uniquement en absence de traitement alcalin de l'extrait gangliosidique.

14. Etude de la distribution du ganglioside GD2 et de sa forme O-acétylée dans le système nerveux et sur des tissus tumoraux

Les échantillons de tumeurs (glioblastome, neuroblastome mélanome de cancer pulmonaire) ont été obtenus à partir d'exérèse chirurgicale. Les échantillons de nerfs périphériques humains ont été prélevés sur le rameau sensitif distal du nerf musculo-cutané, branche latérale du nerf péronier. Il s'agit de prélèvements diagnostiques de neuropathie périphérique ou pour des affections de la corne antérieure (nerfs sensitifs normaux) ayant posé des problèmes diagnostiques. Un volume de tissus ne dépassant pas les 0,5 cm3 est prélevé et congelé dans de l'isopentane refroidi à la température de l'azote liquide. Après 60 secondes, l'échantillon est retiré et transféré dans un tube de congélation préalablement refroifi à -70°C. Des coupes de 10µm de tissu congelé sont réalisées à l'aide d'un cryostat. Les coupes sont recueillies sur des lames en verre Suoerfrost Gold + (VWR). Les coupes sont séchées à l'air pendant 3 minutes puis fixées dans de l'acétone pendant 10 minutes et de nouveau séchées à l'air. Les coupes sont alors conservées à -20°C jusqu'à la réalisation de l'analyse immmunohistochimique. L'immunocoloration des échantillons tissulaires a été réalisée en utilisant les AcM de souris primaires suivants :

Anticorps primaire spécifique du GD2 :

AcM 10B8 (IgG3, kappa) spécifique du GD2
AcM 8B6 (IgG3, kappa) spécifique du GD2-O-Ac

Anticorps primaire servant de réactif de témoin négatif: AcM MCA2063 (IgG3, kappa) spécifique du DNP (Serotec France, Cergy Saint Christophe, Rance).

La révélation de la fixation de ces anticorps sur les échantillons tissulaires testés a été effectuée à l'aide de la trousse DakoCytomation Envision + system, Peroxydase HRP (Dako, Glostrup, Danemark) pour un usage avec les anticorps primaires de la souris selon les recommandations du fournisseur. Les échantillons sont ensuite montés entre lames et lamelles avec um milieu de montage aqueux Aquadex (VWR). La recherche du marquage est alors réalisée par analyse en microscopie optique à l'aide d'un microscope optique au grossissement 100 et 400. Des microphotographies numériques de trois champs microscopiques choisis au hasard par échantillons analysés.

Les échantillons tumoraux, provenant de patients différents, ont tous présenté un marquage positif, membranaire et cytoplasmique, avec les anticorps 10B8 et 8B6 alors que les cellules saines n'ont pas été marquées par l'un ou l'autre de ces anticorps. Aucun marquage n'a été détecté avec l'anticorps MCA2063 anti-DNP.

Les échantillons de nerfs examinés ont tous présenté un marquage au niveau d'internodes de fibres myélinisées avec l'anticorps 10B8 alors que les axones et les fibroblastes ne présentaient pas de marquage. Ce marquage n'est pas retrouvé ou est difficilement détectable avec l'anticorps 8B6. Aucun marquage n'a été mis en évidence avec l'anticorps MCA2063.

15. Etude de la cytotoxicité des anticorps anti-GD2-O-acétylé 8B6 et KM8B6

(a). Préparation de la suspension de cellules cibles.

$1 \times 10^6$ cellules de neuroblastome humain IMR32 cultivées dans du milieu RPMI sont incubées en présence de 1,85 MBq de $Na_2{}^{51}CrO_4$ pendant 1 heure à 37°C. Les cellules sont ensuite lavées trois fois par du RPMI et centrifugation avant d'être resuspendues dans du RPMI et incubées à 4°C pendant 30 minutes afin de mesurer le relargage spontané de la substance radioactive.

Après une dernière centrifugation, les cellules sont reprises dans 5 mL de RPMI afin d'ajuster la concentration à $2 \times 10^5$ cellules/mL.

(b). Préparation des cellules effectrices

Du sang humain à été prélevé sur des donneurs volontaires sur des tubes à prélèvement sanguin contenant de l'héparine. Les leucocytes périphériques du sang ont été séparés du sang total sur un gradient de Ficoll® par centrifugation (1 800 x g pendant 30 minutes). Les cellules obtenues sont centrifugées trois fois dans du RPMI à 1500 x g pour lavage et re-suspendues dans du RPMI afin d'obtenir une concentration en cellules de $5 \times 10^6$ cellules/mL.

(c). Mesure de l'activité ADCC

Dans chacun des puits de plaque de microtitration à 96 puits en fond en U, obtenue auprès de la société Falcon, 50 µL de suspension de cellules cibles, obtenues en a., sont déposés. 100 µL de la suspension de cellules effectrices, obtenue en b., sont ensuite ajoutés soit 50 000 cellules par puit. Le ratio de cellules cibles sur le nombre de cellules effectrices est de 1/50. L'anticorps 8B6, et l'anticorps modifié artificiellement KM8B6 et le rituxan®, anticorps hommesouris IgG1, kappa, anti-CD20 utilisé comme contrôle négatif) sont ensuite ajoutés dans chaque puit à la concentration de 1 µg/mL ou de 10 µg/mL. Le mélange est incubés 4 heures à 37°C. Après centrifugation, la plaque est centrifugée et la quantité de $^{51}Cr$ présente dans le surnageant est mesurée à l'aide d'un compteur y. La quantité totale de $^{51}Cr$ libérée est mesurée selon la même procédure en ajoutant du milieu sans anticorps, et une solution de soude 5 N à la place de la suspension de cellules effectrices. L'ADCC est alors calculée en utilisant la formule suivante :

$$\text{\% activité d'ADCC} = (^{51}\text{Cr présent dans le surnageant} - {}^{51}\text{Cr libéré spontanément})/ ({}^{51}\text{Cr total} - {}^{51}\text{Cr libéré spontanément})$$

16. Activité tumorale de l'anticorps 8B6 dans un modèle syngénique de lymphome de souris.

L'activité anti-tumorale de l'anticorps 8B6 a été déterminée dans le modèle de greffe sous-cutanée syngénique du lymphome T de souris EL-4, qui exprime l'antigène GD2, chez la souris de souche C57BL/6 (Zhang H, Zhang S, Cheung NK, Ragupathi G, Livingston PO. Antibodies against GD2 ganglioside can eradicate syngeneic cancer micrometastases. Cancer Res. 1998, 58: 2844-9). Ces cellules EL4 expriment également l'atigène GD2-O-acétyl Vingt quatre souris élevées en animalerie agréée de niveau A1 ont reçu une injection sous-cutanée de $20 \times 10^4$ cellules EL-4 en suspension dans du PBS, à l'âge de 12 semaines. Deux lots de 12 souris ont été constitués. Les souris du lot A ont reçu 70 $\mu$g d'AcM 8B6 en solution dans 200 $\mu$L de solution tampon PBS par voie i.v., tous les 3 jours à partir du premier jour après l'injection des cellules EL4 et jusqu'au 21$^{\text{ème}}$ jour. Les souris du lot B ont reçu, selon les mêmes modalités, uniquement 200 $\mu$L de solution PBS. Le volume des tumeurs a ensuite été mesuré tous les deux jours. Le volume tumoral a été estimé grâce à la formule suivante : volume (mm$^3$) = longueur (mm) x largeur$^2$ (mm) x 0,5 (Zeng G, Li DD, Gao L, Birkle S, Bieberich E, Tokuda A, Yu RK.Alteration of ganglioside composition by stable transfection with antisense vectors against GD3-synthase gene expression. Biochemistry 1999 38: 8762-9). Les souris présentant un volume $\geq$ 3.000 mm$^3$ sont sacrifiés.

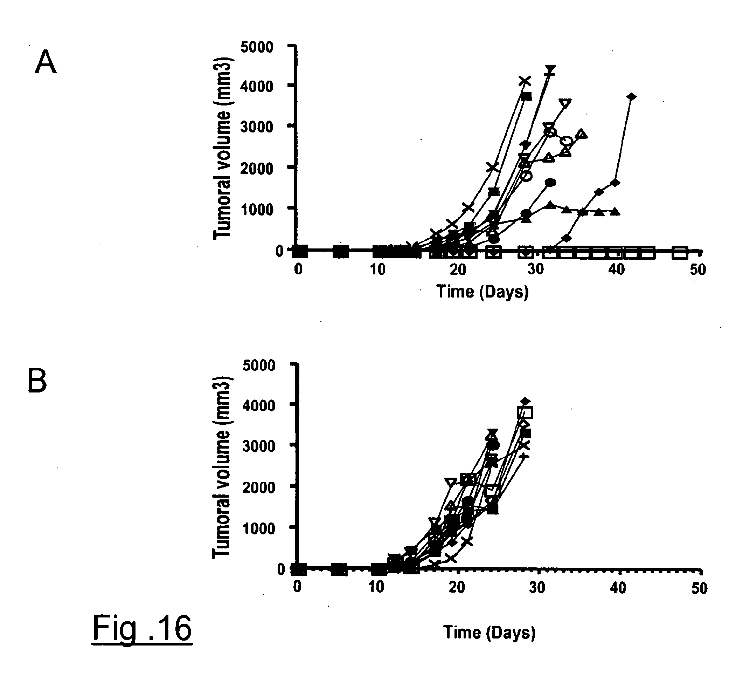

Les résultats obtenus sont présentés dans la Figure 16. Le lot B recevant le PBS par voie i.v., correspond au lot témoin non traité (Figure 16, panneau B). Les tumeurs commencent à être détectable 10 jours après inoculation et montrent ensuite une croissance exponentielle. Vingt jours après inoculation, toutes les souris sauf deux ont une tumeur de plus de 1000 mm$^3$, Toutes les souris ont été sacrifiées avant le jour 30 suivant les règles de l'expérimentation animale car leur tumeur dépassait 3000 mm$^3$. Chez les souris du lot A, traitées avec l'anticorps 8B6 (Figure 16, panneau A), un retard d'apparition des tumeurs est observé. De plus, la croissance tumorale est ralentie chez les souris ayant reçu cet anticorps. Vingt jours après l'inoculation, aucune des souris traitées n'a une tumeur de plus de 1000 mmm$^3$. Au bout de 30 jours, 58 % des souris traitées avec l'AcM 8B6 sont survivantes alors que toutes les souris non traitées sont mortes. Au bout de 40 jours, 25 % des souris traitées sont encore en vie et au 50$^{\text{ème}}$ jour, 8 % des souris sont encore en vie et ne présentent pas de tumeurs papables et sont considérées comme guéries.

**Revendications**

1. Anticorps reconnaissant le anglioside GD2-O-acétylé et ne reconnaissant pas le ganglioside GD2 pour l'utilisation dans le traitement des cancers exprimant le GD2-O-acétylé, notamment les tumeurs d'origine neuroectodermique exprimant le GD2 et sa forme O-acétylée.

2. Anticorps pour l'utilisation selon la revendication 1 **caractérisée en ce que** l'anticorps ne reconnaissant que la forme O-acétylée du ganglioside GD2 est une IgG kappa d'affinité supérieure à $10^7$ litre/mole pour le GD2-O-acétylé et d'affinité au moins dix fois plus faible pour le GD2 lui-même

3. Anticorps pour l'utilisation selon la revendication 1 **caractérisée en ce que** l'anticorps ne reconnaissant que la forme O-acétylée du ganglioside GD2 est un anticorps monoclonal ou fragment de cet anticorps.

4. Anticorps pour l'utilisation selon la revendication 2 **caractérisée en ce que** certains amino-acides de l'anticorps ne reconnaissant que la forme O-acétylée du ganglioside GD2 ont été remplacés par d'autres en utilisant les techniques de génétique moléculaire connues de l'homme de l'art, notamment pour modifier les propriétés de anticorps orignal en particulier pour diminuer son immunogénicité, ou pour augmenter son activité toxique ou encore pour accélérer ou ralentir son élimination après injection.

5. Anticorps pour l'utilisation selon la revendication 4, **caractérisée en ce que** ledit anticorps présente un pouvoir cytotoxique augmenté et **en ce qu'**il est peu fucosylé.

6. Anticorps pour l'utilisation selon la revendication 2 **caractérisée en ce que** l'anticorps ne reconnaissant que la forme O-acétylée du ganglioside GD2 est l'anticorps 8B6 dont les régions déterminant la complémentarité de la région variable de la chaîne H ont comme séquences d'aminoacides celles représentées dans SEQ ID NO:2, SEQ ID NO:3 et SEQ ID NO:4 et les régions déterminant la complémentarité de la région variable de la chaîne L ont comme séquences d'aminoacides celles représentées dans SEQ ID NO:6, SEQ ID NO:7 et SEQ ID NO:8.

7. Anticorps pour l'utilisation selon la revendication 2 **caractérisée en ce que** l'anticorps ne reconnaissant que la forme O-acétylée du ganglioside GD2 est un anticorps chimérique ou fragment de celui-ci.

8. Anticorps pour l'utilisation selon la revendication 2 **caractérisée en ce que** l'anticorps ne reconnaissant que la forme O-acétylée du ganglioside GD2 est un anticorps humanisé ou fragment de celui-ci.

9. Anticorps pour l'utilisation selon l'une des revendications 6, 7 ou 8 **caractérisée en ce que** l'anticorps ne reconnaissant que la forme O-acétylée du ganglioside GD2 est un anticorps chimérique ou humanisé dont les régions déterminant la complémentarité de la région variable de la chaîne H ont comme séquences d'aminoacides celles représentées dans SEQ ID NO:2, SEQ ID NO:3 et SEQ ID NO:4 et les régions déterminant la complémentarité de la région variable de la chaîne L ont comme séquences d'aminoacides celles représentées dans SEQ ID NO:6, SEQ ID NO:7 et SEQ ID NO:8.

10. Anticorps pour l'utilisation selon les revendications 1 à 9 **caractérisée en ce que** lesdits cancers sont des neuroblastomes, des mélanomes, des glioblastomes ou des cancers pulmonaires à petites cellules.

**11.** Anticorps pour l'utilisation selon les revendications 1 à 9 **caractérisée en ce que** l'anticorps ou fragment de celui-ci est couplé avec une molécule X, où X est une molécule toxique, un médicament, une pro-drogue, ou un deuxième anticorps quelle que soit sa spécificité.

**12.** Anticorps pour l'utilisation selon la revendication 11 **caractérisée en ce que** ladite molécule toxique est une molécule toxique chimique, biologique ou radioactive, ladite molécule étant destinée à détruire des cellules tumorales exprimant le ganglioside GD2-O-acétylé.

**13.** Anticorps pour l'utilisation selon l'une quelconque des revendications 1 à 9 **caractérisée en ce que** l'anticorps est muté au niveau de sa région Fc par l'adjonction de sucres, modulant ainsi l'activation des cellules immunitaires et des molécules du système du complément.

**Claims**

**1.** Antibody recognizing O-acetylated GD2 ganglioside and not recognizing GD2 ganglioside for use in the treatment of cancers expressing O-acetylated GD2, in particular tumors of neuroectodermal origin expressing GD2 and its O-acetylated form.

**2.** Antibody for the use according to claim 1 **characterized in that** the antibody only recognizing the O-acetylated form of GD2 ganglioside is a kappa-IgG having an affinity of more than $10^7$ liter/mol for O-acetylated GD2 and having an affinity of at least ten times lower for GD2 itself.

**3.** Antibody for the use according to claim 1 **characterized in that** the antibody only recognizing the O-acetylated form of GD2 ganglioside is a monoclonal antibody or a fragment of said antibody.

**4.** Antibody for the use according to claim 2 **characterized in that** some amino acids of the antibody only recognizing the O-acetylated form of GD2 ganglioside have been replaced with others using molecular genetic techniques known by those skilled in the art, in particular for modifying the properties of the original antibody, especially for decreasing its immunogenicity, or for increasing its toxic activity or even for speeding up or slowing down its clearance after injection.

**5.** Antibody for the use according to claim 4, **characterized in that** said antibody has an increased cytotoxic activity and is low-fucosylated.

**6.** Antibody for the use according to claim 2 **characterized in that** said antibody only recognizing the O-acetylated form of GD2 ganglioside is 8B6 antibody having complementarity-determining regions of the H chain variable region comprising amino acid sequences represented in SEQ ID NO:2, SEQ ID NO:3 and SEQ ID NO:4 and having complementarity-determining regions of the L chain variable region comprising amino acid sequences represented in SEQ ID NO:6, SEQ ID NO:7 and SEQ ID NO:8.

**7.** Antibody for the use according to claim 2 **characterized in that** the antibody only recognizing the O-acetylated form of GD2 ganglioside is a chimeric antibody or a fragment thereof.

**8.** Antibody for the use according to claim 2 **characterized in that** the antibody only recognizing the O-acetylated form of GD2 ganglioside is a humanized antibody or a fragment thereof.

**9.** Antibody for the use according to claim 6,7 or 8 **characterized in that** the antibody only recognizing the O-acetylated form of GD2 ganglioside is a chimeric or humanized antibody having complementarity-determining regions of the H chain variable region comprising amino acid sequences represented in SEQ ID NO:2, SEQ ID NO:3 and SEQ ID NO:4 and having complementarity-determining regions of the L chain variable region comprising amino acid sequences represented in SEQ ID NO:6, SEQ ID NO:7 and SEQ ID NO:8.

**10.** Antibody for the use according to claims 1 to 9 **characterized in that** said cancers are neuroblastomas, melanomas, glioblastomas, or small cell lung cancers.

**11.** Antibody for the use according to claims 1 to 9 **characterized in that** the antibody or a fragment thereof is coupled with a molecule X, where X is a toxic molecule, a drug, a pro-drug, or a second antibody irrespective of its specificity.

**12.** Antibody for the use according to claim 11 **characterized in that** said toxic molecule is a toxic chemical, biological or radioactive molecule, said molecule being used to kill tumor cells expressing O-acetylated GD2 ganglioside.

**13.** Antibody for the use according to claims 1 to 9 **characterized in that** the antibody is mutated at its Fc region by the adjunction of sugars, thus modulating activation of the immune cells and of the complement system molecules.

**Patentansprüche**

**1.** Antikörper, der das O-acetylierte GD2-Gangliosid erkennt und das GD2-Gangliosid nicht erkennt, für die Verwendung bei der Behandlung von Krebserkrankungen, die das O-acetylierte GD2 exprimieren, insbesondere Tumoren neuroektodermalen Ursprungs, die GD2 und seine O-acetylierte Form exprimieren.

**2.** Antikörper zur Verwendung nach Anspruch 1, **dadurch gekennzeichnet, dass** es sich bei dem Antikörper, der nur die O-acetylierte Form des GD2-Gangliosids erkennt, um ein IgG des Typs kappa mit einer Affinität von oberhalb $10^7$ Liter/Mol für das O-acetylierte GD2 und mit einer mindestens zehnmal schwächeren Affinität für das GD2 selbst handelt.

**3.** Antikörper zur Verwendung nach Anspruch 1, **dadurch gekennzeichnet, dass** es sich bei dem Antikörper, der nur die O-acetylierte Form des GD2-Gangliosids erkennt, um einen monoklonalen Antikörper oder ein Fragment dieses Antikörpers handelt.

**4.** Antikörper zur Verwendung nach Anspruch 2, **dadurch gekennzeichnet, dass** bestimmte Aminosäuren des Antikörpers, der nur die O-acetylierte Form des GD2-Gangliosids erkennt, mit Hilfe von molekulargenetischen Techniken, mit denen der Fachmann vertraut ist, durch andere Aminosäuren ersetzt worden sind, insbesondere um die Eigenschaften des ursprünglichen Antikörpers zu modifizieren, ganz besonders um seine Immunogenität zu verringern oder um seine toxische Aktivität zu erhöhen oder auch um seine Elimination nach der Injektion zu beschleunigen oder zu verlangsamen.

**5.** Antikörper zur Verwendung nach Anspruch 4, **dadurch gekennzeichnet, dass** der Antikörper eine erhöhte zytotoxische Wirkung aufweist und dass er wenig fucosyliert ist.

**6.** Antikörper zur Verwendung nach Anspruch 2, **dadurch gekennzeichnet, dass** es sich bei dem Antikörper, der nur die O-acetylierte Form des GD2-Gangliosids erkennt, um den Antikörper 8B6 handelt, dessen komplementaritätsdeterminierende Regionen der variablen Region der H-Kette als Aminosäuresequenzen die Sequenzen gemäß SEQ ID NO: 2, SEQ ID NO: 3 und SEQ ID NO: 4 aufweisen und die komplementaritätsdeterminierenden Regionen der variablen Region der L-Kette als Aminosäuresequenzen die Sequenzen gemäß SEQ ID NO: 6, SEQ ID NO: 7 und SEQ ID NO: 8 aufweisen.

**7.** Antikörper zur Verwendung nach Anspruch 2, **dadurch gekennzeichnet, dass** es sich bei dem Antikörper, der nur die O-acetylierte Form des GD2-Gangliosids erkennt, um einen chimären Antikörper oder ein Fragment dieses Antikörpers handelt.

**8.** Antikörper zur Verwendung nach Anspruch 2, **dadurch gekennzeichnet, dass** es sich bei dem Antikörper, der nur die O-acetylierte Form des GD2-Gangliosids erkennt, um einen humanisierten Antikörper oder ein Fragment dieses Antikörpers handelt.

**9.** Antikörper zur Verwendung nach einem der Ansprüche 6, 7 oder 8, **dadurch gekennzeichnet, dass** es sich bei dem Antikörper, der nur die O-acetylierte Form des GD2-Gangliosids erkennt, um einen chimären oder humanisierten Antikörper handelt, dessen komplementaritätsdeterminierende Regionen der variablen Region der H-Kette als Aminosäuresequenzen die Sequenzen gemäß SEQ ID NO: 2, SEQ ID NO: 3 und SEQ ID NO: 4 aufweisen und die komplementaritätsdeterminierenden Regionen der variablen Region der L-Kette als Aminosäuresequenzen die Sequenzen gemäß SEQ ID NO: 6, SEQ ID NO: 7 und SEQ ID NO: 8 aufweisen.

**10.** Antikörper zur Verwendung nach einem der Ansprüche 1 bis 9, **dadurch gekennzeichnet, dass** es sich bei den Krebserkrankungen um Neuroblastome, Melanome, Glioblastome oder kleinzellige Lungenkarzinome handelt.

**11.** Antikörper zur Verwendung nach einem der Ansprüche 1 bis 9, **dadurch gekennzeichnet, dass** der Antikörper oder dessen Fragment mit einem Molekül X gekoppelt ist, wobei es sich bei X um ein toxisches Molekül, einen Arzneistoff, ein Prodrug oder einen zweiten Antikörper mit beliebiger Spezifität handelt.

12. Antikörper zur Verwendung nach Anspruch 11, **dadurch gekennzeichnet, dass** es sich bei dem toxischen Molekül um ein chemisches, biologisches oder radioaktives toxisches Molekül handelt, wobei das Molekül dazu dient, um Tumorzellen, die das O-acetylierte GD2-Gangliosid exprimieren, abzutöten.

13. Antikörper zur Verwendung nach einem der Ansprüche 1 bis 9, **dadurch gekennzeichnet, dass** der Antikörper in seiner Fc-Region durch Hinzufügen von Zuckern mutiert ist, wodurch die Aktivierung der Immunzellen und der Moleküle des Komplement-Systems moduliert wird.

Fig. 1

Fig. 2

Fig. 3

Fig. 5

```
BamH I
  |                  - 19                                    -11
GGA TCC GCC ACC ATG AAG TTG CCT GTT AGG CTG TTG GTG CTG ATG TTC TGG ATT CCT GGT TCC AGC
            M   K   L   P   V   R   L   L   V   L   M   F   W   I   P   G   S   S
            | → Peptide signal


      1
AGT GAT GTT GTG ATG ACC CAA ACT CCA CTC TCC CTG CCT GTC AGT CTT GGA GAT CAA GCC TCA ATC
S   D   V   V   M   T   Q   T   P   L   S   L   P   V   S   L   G   D   Q   A   S   I
    | → FR1


                                      31                                    41
TCT TGC AGA TCT AGT CAG AGC CTT CTA AAA AAT AAT GGA AAC ACC TTT TTA CAT TGG TAC CTG CAG
S   C   R   S   S   Q   S   L   L   K   N   N   G   N   T   F   L   H   W   Y   L   Q
                    | → CDR1                                     | → FR2


                              51                              61
AAG TCA GGC CAG TCT CCA AAG CTC CTT ATC TAC AAA GTT TCC AAC CGA CTT TCT GGG GTC CCA GAC
K   S   G   Q   S   P   K   L   L   I   Y   K   V   S   N   R   L   S   G   V   P   D
                                      | → CDR2    | → FR2


                      71                              81
AGG TTC AGT GGC AGT GGA TCA GGG ACA TAT TTC ACA CTC AAG ATC AGC AGA GTG GAG GCT GAG GAT
R   F   S   G   S   G   S   G   T   Y   F   T   L   K   I   S   R   V   E   A   E   D


                                                                          Xho I
                      91                              101                    |
CTG GGA GTT TAT TTC TGC TCT CAA AGT ACA CAT ATT CCG TAC ACA TTC GGA GGG GGG ACC AAG CTC
L   G   V   Y   F   C   S   Q   S   T   H   I   P   Y   T   F   G   G   G   T   K   L
                        | → CDR 3                             | → FR4
      111                                            121                            131
GAG CTG AAA CGA ACT GTG GCT GCA CCA TCT GTC TTC ATC TTC CCG CCA TCT GAT GAG CAG TTG AAA
E   L   K   R   T   V   A   A   P   S   V   F   I   F   P   P   S   D   E   Q   L   K
                | → C kappa humain


                                      141                                    151
TCT GGA ACT GCC TCT GTT GTG TGC CTG CTG AAT AAC TTC TAT CCC AGA GAG GCC AAA GTA CAG TGG
S   G   T   A   S   V   V   C   L   L   N   N   F   Y   P   R   E   A   K   V   Q   W


                              161                              171
AAG GTG GAT AAC GCC CTC CAA TCG GGT AAC TCC CAG GAG AGT GTC ACA GAG CAG GAC AGC AAG GAC
K   V   D   N   A   L   Q   S   G   N   S   Q   E   S   V   T   E   Q   D   S   K   D


                      181                              191
AGC ACC TAC AGC CTC AGC AGC ACC CTG ACG CTG AGC AAA GCA GAC TAC GAG AAA CAC AAA GTC TAC
S   T   Y   S   L   S   S   T   L   T   L   S   K   A   D   Y   E   K   H   K   V   Y


              201                              211                            219
GCC TGC GAA GTC ACC CAT CAG GGC CTG AGC TCG CCC GTC ACA AAG AGC TTC AAC AGG GGA GAG TGT
A   C   E   V   T   H   Q   G   L   S   S   P   V   T   K   S   F   N   R   G   E   C


      Xba I
        |
TAA TCT AGA
 *
```

Fig. 4

Fig. 6

Fig. 7

Fig. 8

BamH I

G GAT CCG GCC ACC ATG AAG TTG TGG CTG AAC TGG ATT TTC CTT GTA ACA CTT TTA AAT GGT TTC CAG TGT GAG GTG AAA CTG GTG GAG TCT GGA GGA GGC TTG GTG CTG CCT GGG GAT
  M   K   L   W   L   N   W   I   F   L   V   T   L   L   N   G   F   Q   C   E   V   K   L   V   E   S   G   G   G   L   V   L   P   G   D
  |-- Peptido signal                                                              |-- FR1

TCT CTG AGA CTC TCC TGT GCA ACT TCT GAG TTC ACC TTC ACT GAT TAC TAC ATG ACT TGG GTC CGC CAG CCT CCA AGA AAG GCA CTT GAG TGG TTG GGT TTT ATT AGA AAC AGA GCT AAT
  S   L   R   L   S   C   A   T   S   E   F   T   F   T   D   Y   Y   M   T   W   V   R   Q   P   P   R   K   A   L   E   W   L   G   F   I   R   N   R   A   N
                      |-- CDR1                              |-- FR2                                                                  |-- CDR2

GGT TAC ACA ACA GAG TAC AAT CCA TCT GTG AAG GGT CGG TTC ACC ATT TCC AGA GAT AAT TCC CAA AGC ATC CTC TAT CTT CAA ATG AAC ACC CTG AGA ACT GAG GAC AGT GCC ACT TAT
  G   Y   T   T   E   Y   N   P   S   V   K   G   R   F   T   I   S   R   D   N   S   Q   S   I   L   Y   L   Q   M   N   T   L   R   T   E   D   S   A   T   Y
          |-- FR3

Nho I

TAC TGT GCA AGA GTC TCT AAC TGG GCC TTT GAC TAC TGG GGC CAA GGC ACC ACT CTC ACA GTC TCC TCA GCT AGC ACC AAG GGC CCA TCG GTC TTC CCC CTG GCA CCC TCC TCC AAG AGC
  Y   C   A   R   V   S   N   W   A   F   D   Y   W   G   Q   G   T   T   L   T   V   S   S   A   S   T   K   G   P   S   V   F   P   L   A   P   S   S   K   S
          |-- CDR3                              |-- FR4                              |-- C gamma 1 humain

ACC TCT GGG GGC ACA GCG GCC CTG GGC TGC CTG GTC AAG GAC TAC TTC CCC GAA CCG GTG ACG GTG TCG TGG AAC TCA GGC GCC CTG ACC AGC GGC GTG CAC ACC TTC CCG GCT GTC CTA
  T   S   G   G   T   A   A   L   G   C   L   V   K   D   Y   F   P   E   P   V   T   V   S   W   N   S   G   A   L   T   S   G   V   H   T   F   P   A   V   L

CAG TCC TCA AGA CTC TAC TCC CTC AGC AGC GTG GTG ACC GTG CCC TCC AGC AGC TTG GGC ACC CAG ACC TAC ATC TGC AAC GTG AAT CAC AAG CCC AGC AAC ACC AAG GTG GAC AAG AGA
  Q   S   S   R   L   Y   S   L   S   S   V   V   T   V   P   S   S   S   L   G   T   Q   T   Y   I   C   N   V   N   H   K   P   S   N   T   K   V   D   K   R

GTT GAG CCC AAA TCT TGT GAC AAA ACT CAC ACA TGC CCA CCG TGC CCA GCA CCT GAA CTC CTG GGG GGA CCG TCA GTC TTC CTC TTC CCC CCA AAA CCC AAG GAC ACC CTC ATG ATC TCC
  V   E   P   K   S   C   D   K   T   H   T   C   P   P   C   P   A   P   E   L   L   G   G   P   S   V   F   L   F   P   P   K   P   K   D   T   L   M   I   S

CGG ACC CCT GAG GTC ACA TGC GTG GTG GTG GAC GTG AGC CAC GAA GAC CCT GAG GTC AAG TTC AAC TGG TAC GTG GAC GGC GTG GAG GTG CAT AAT GCC AAG ACA AAG CCG CGG GAG GAG
  R   T   P   E   V   T   C   V   V   V   D   V   S   H   E   D   P   E   V   K   F   N   W   Y   V   D   G   V   E   V   H   N   A   K   T   K   P   R   E   E

CAG TAC AAC AGC ACG TAC CGT GTG GTC AGC GTC CTC ACC GTC CTG CAC CAG GAC TGG CTG AAT GGC AAG GAG TAC AAG TGC AAG GTC TCC AAC AAA GCC CTC CCA GCC CCC ATC GAG AAA
  Q   Y   N   S   T   Y   R   V   V   S   V   L   T   V   L   H   Q   D   W   L   N   G   K   E   Y   K   C   K   V   S   N   K   A   L   P   A   P   I   E   K

ACC ATC TCC AAA GCC AAA GGG CAG CCC CGA GAA CCA CAG GTG TAC ACC CTG CCC CCA TCC CGG GAG GAG ATG ACC AAG AAC CAG GTC AGC CTG ACC TGC CTG GTC AAA GGC TTC TAT CCC
  T   I   S   K   A   K   G   Q   P   R   E   P   Q   V   Y   T   L   P   P   S   R   E   E   M   T   K   N   Q   V   S   L   T   C   L   V   K   G   F   Y   P

AGC GAC ATC GCC GTG GAG TGG GAG AGC AAT GGG CAG CCG GAG AAC AAC TAC AAG ACC ACG CCT CCC GTG CTG GAC TCC GAC GGC TCC TTC TTC CTC TAT AGC AAG CTC ACC GTG GAC AAG
  S   D   I   A   V   E   W   E   S   N   G   Q   P   E   N   N   Y   K   T   T   P   P   V   L   D   S   D   G   S   F   F   L   Y   S   K   L   T   V   D   K

Xho I

AGC AGG TGG CAG CAG GGG AAC GTC TTC TCA TGC TCC GTG ATG CAT GAG GCT CTG CAC AAC CAC TAC ACG CAG AAG AGC CTC TCC CTG TCT CCG GGT AAA TAA TCT AGA
  S   R   W   Q   Q   G   N   V   F   S   C   S   V   M   H   E   A   L   H   N   H   Y   T   Q   K   S   L   S   L   S   P   G   K   *

<u>Fig. 9</u>

Fig. 10

KM8B6                8B6

Nombre de cellule relatif

IMR32 (GD2+)

Neuro 2A (GD2-)

Intensité de fluorescence

Fig. 11

Fig. 12

Fig. 13

Fig. 14

| Anticoprs | Concentration | | | |
|---|---|---|---|---|
| | 0 µg/mL | 0,1 µg/mL | 1 µg/mL | 10 µg/mL |
| 8B6 | 0 % | 10 % | 20 % | 30 % |
| KM8B6 | 0 % | 16 % | 23 % | 44 % |
| AcM non spécifique* | 0 % | 0 % | 0 % | 0 % |

## Fig. 15

Fig.16

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **J.N.YE et al.** *Int.J. Cancer,* 1992, vol. 50, 197-201
- **Cerato et al.** Variable Region Gene Segments of Nine Monoclonal Antibodies Specific to Disialoganglisosides (GD2,GD3) and their O-Actylated Derivatives. *Hybridoma,* 1997, vol. 16 (4), 307-316
- **Cerato et al.** Variable Region Gene Segments of Nine Monoclonal Antibodies Specific to Disialoganglisosides (GD2, GD3) and their O-Actylated Derivatives. *Hybridoma,* 1997, vol. 16 (4), 307-316
- **Zhang H ; Zhang S ; Cheung NK ; Ragupathi G ; Livingston PO.** Antibodies against GD2 ganglioside can eradicate syngeneic cancer micrometastases. *Cancer Res.,* 1998, vol. 58, 2844-9
- **Zeng G ; Li DD ; Gao L ; Birkle S ; Bieberich E ; Tokuda A ; Yu RK.** Alteration of ganglioside composition by stable transfection with antisense vectors against GD3-synthase gene expression. *Biochemistry,* 1999, vol. 38, 8762-9

SEQUENCE LISTING

<110> université de Nantes

<120> utilisation d'anticorps monoclonaux spécifiques du ganglioside GD2-O-acétylé dans le traitement de certains cancers

<130> R11732FR

<160> 8

<170> PatentIn version 3.3

<210> 1
<211> 137
<212> PRT
<213> souris

<400> 1

```
Met Lys Leu Trp Leu Asn Trp Ile Phe Leu Val Thr Leu Leu Asn Gly
1                5                    10                  15

Phe Gln Cys Glu Val Lys Leu Val Glu Ser Gly Gly Gly Leu Val Leu
            20              25                  30

Pro Gly Asp Ser Leu Arg Leu Ser Cys Ala Thr Ser Glu Phe Thr Phe
        35              40                  45

Thr Asp Tyr Tyr Met Thr Trp Val Arg Gln Pro Pro Arg Lys Ala Leu
    50              55                  60

Glu Trp Leu Gly Phe Ile Arg Asn Arg Ala Asn Gly Tyr Thr Thr Glu
65              70                  75                      80

Tyr Asn Pro Ser Val Lys Gly Arg Phe Thr Ile Ser Arg Asp Asn Ser
            85                  90                  95

Gln Ile Leu Tyr Leu Gln Met Asn Thr Leu Arg Thr Glu Asp Ser Ala
            100                 105                 110

Thr Tyr Tyr Cys Ala Arg Val Ser Asn Trp Ala Phe Asp Tyr Trp Gly
        115                 120                 125

Gln Thr Thr Leu Thr Val Ser Ser Ala
    130                 135
```

<210> 2
<211> 8
<212> PRT
<213> souris

<400> 2

```
Glu Phe Thr Phe Thr Asp Tyr Tyr
1                5
```

<210> 3
<211> 10
<212> PRT
<213> souris

<400> 3

```
Ile Arg Asn Arg Ala Asn Gly Tyr Thr Thr
1                5                    10
```

<210> 4
<211> 10
<212> PRT
<213> souris

<400> 4

24

```
                      Ala Arg Val Ser Asn Trp Ala Phe Asp Tyr
                      1               5                   10
```

<210> 5
<211> 133
<212> PRT
<213> souris

<400> 5

```
        Met Lys Leu Pro Val Arg Leu Leu Val Leu Met Phe Trp Ile Pro Gly
        1           5               10              15

        Ser Ser Ser Asp Val Val Met Thr Gln Thr Pro Leu Ser Leu Pro Val
                    20              25              30

        Ser Leu Gly Asp Gln Ala Ser Ile Ser Cys Arg Ser Ser Gln Ser Leu
                35              40              45

        Leu Lys Asn Asn Gly Asn Thr Phe Leu His Trp Tyr Leu Gln Lys Ser
            50              55              60

        Gly Gln Ser Pro Lys Leu Leu Ile Tyr Lys Val Ser Asn Arg Leu Ser
        65              70              75              80

        Gly Val Pro Asp Arg Phe Ser Gly Ser Gly Ser Gly Thr Tyr Phe Thr
                        85              90              95

        Leu Lys Ile Ser Arg Val Glu Ala Glu Asp Leu Gly Val Tyr Phe Cys
                    100             105             110

        Ser Gln Ser Thr His Ile Pro Tyr Thr Phe Gly Gly Gly Thr Lys Leu
                    115             120             125

        Glu Leu Lys Arg Thr
                    130
```

<210> 6
<211> 12
<212> PRT
<213> souris

<400> 6

```
        Gln Ser Leu Leu Lys Asn Asn Gly Asn Thr Phe Leu
        1           5               10
```

<210> 7
<211> 3
<212> PRT
<213> souris

<400> 7

```
Lys Val Ser
1
```

<210> 8
<211> 9
<212> PRT
<213> souris

<400> 8

```
Ser Gln Ser Thr His Ile Pro Tyr Thr
1                   5
```